# EUROPEAN PATENT APPLICATION

(11) **EP 4 702 970 A1**
(43) Date of publication of application: **04.03.2026**
(21) Application number: 24796925.6
(22) Date of filing: 19.04.2024
(51) Int. Cl.: A61K 8/898, A61K 8/06, A61K 8/19, A61Q 1/00

(54) **COSMETIC CONTAINING AMINO ALCOHOL-MODIFIED SILICONE**

(30) Priority: 25.04.2023 JP 2023071593
(71) Applicant: SHIN-ETSU CHEMICAL CO., LTD., Tokyo 1000005 (JP)
(72) Inventor: IMAI Taro, Annaka-shi, Gunma 379-0224 (JP); KONISHI Masayuki, Tokyo 100-0005 (JP)
(74) Representative: Mewburn Ellis LLP
(86) International application number: PCT/JP2024/015494
(87) International publication number: WO 2024/225178

(57) **Abstract**

Provided is a water-in-oil cosmetic comprising
(a) an amino alcohol-modified silicone represented by formula (1) 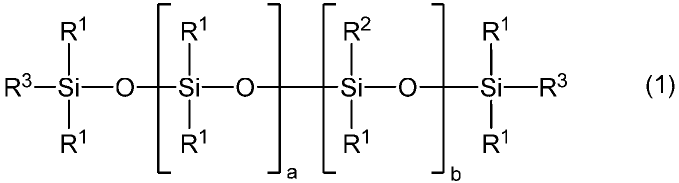 [R¹ is a monovalent hydrocarbon group, R² is a group represented by formula (2) 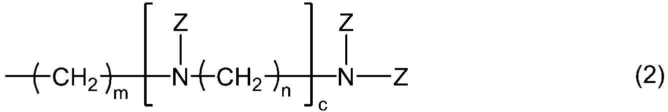 (Z is a group independently selected from a hydrogen atom, -CH₂CH(OH)CH₃,
and -CH(CH₃)CH₂OH, 50 mol% or more, relative to the total number of mol of Z in the molecule, being a group selected from -CH₂CH(OH)CH₃ and -CH(CH₃)CH₂OH. m is an integer of 1-10, and n is an integer of 1-5.)] and
(b) 0.6-1.8 mass% of an inorganic salt.
The cosmetic has a fine emulsified particle size, an excellent feel on use, and excellent viscosity stability.

## Description

### TECHNICAL FIELD

The present invention relates to a cosmetic containing an aminoalcohol-modified silicone. In the present invention, a composition for use in a cosmetic may be written as a cosmetic.

### BACKGROUND ART

Patent Document 1 proposes a method for synthesizing a polyglycerin-modified silicone as a composition by the addition reaction of a polyhydric alcohol-substituted hydrocarbon group having at least two hydroxyl groups and a silicone compound to an organohydrogenpolysiloxane. However, polyglycerin-modified silicones have poor interfacial tension reducing ability, and this fact makes it difficult to reduce the emulsion particle size.

Patent Document 2 discloses a cosmetic that contains an aminoalcohol-modified organopolysiloxane as a silicone surfactant. This patent document also mentions the emulsion stability of the cosmetic including the aminoalcohol-modified organopolysiloxane. However, over-time changes in viscosity are not actually measured and the cosmetic is stable only as a result of the viscosity being increased by the addition of a large amount of silicone gelling agent or bentonite. Furthermore, no consideration is given to the optimal silicone main chain length, and the cosmetic is stabilized only by adding viscosity through the use of a high-viscosity aminoalcohol-modified silicone that is unpleasant to the touch. Patent Document 3 discusses an emulsion composition containing an aminoalcohol-modified organosiloxane. However, this composition is intended for use in, for example, laundry detergents and fabric softeners, and there is no mention of cosmetic use, such as emulsification performance or feeling on use.

### PRIOR ART DOCUMENTS

### PATENT DOCUMENTS

Patent Document 1: JP-A 2002-179798
Patent Document 2: JP-A 2015-113344
Patent Document 3: JP-A 2013-523963

### SUMMARY OF INVENTION

### TECHNICAL PROBLEM

The present invention has been made in view of the circumstances discussed above. It is an object of the present invention to provide a water-in-oil cosmetic that has a small emulsion particle size and is excellent in feeling on use and viscosity stability.

### SOLUTION TO PROBLEM

As a result of extensive studies directed to achieving the above object, the present inventors have found that the problems described above can be solved with a water-in-oil cosmetic that contains a specific organopolysiloxane and a specific amount of an inorganic salt. The present invention has been completed based on the finding.

Thus, the present invention provides the following water-in-oil cosmetics.
1. A water-in-oil cosmetic including:
   (a) an aminoalcohol-modified silicone represented by formula (1) below: 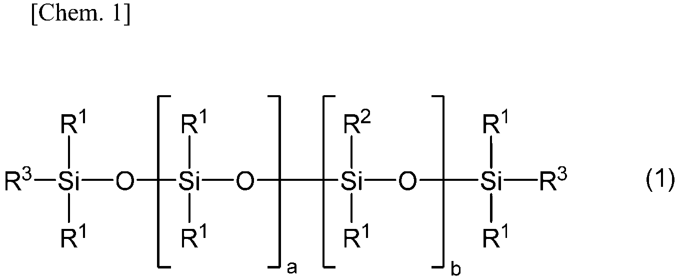 [R¹ independently at each occurrence is a monovalent hydrocarbon group selected from C1-C20 alkyl groups, C6-C15 aryl groups, and C7-C15 aralkyl groups; R² independently at each occurrence is a group represented by formula (2) below: 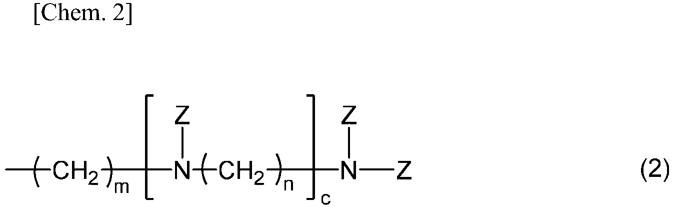 (Z independently at each occurrence is a group selected from hydrogen atom, -CH₂CH(OH)CH₃, and -CH(CH₃)CH₂OH; 50 mol% or more of a number of moles of all Zs in the molecule are groups selected from -CH₂CH(OH)CH₃ and -CH(CH₃)CH₂OH; m is an integer of 1 to 10; and n is an integer of 1 to 5); R³ is R¹ or R²; a is an integer of 1 to 100; b is an integer of 0 to 10; c is an integer of 0 to 4; and when b = 0, at least one of R³s is R²], and
   (b) 0.6 to 1.8 mass% of an inorganic salt.
2. The water-in-oil cosmetic according to 1, wherein in formula (1), R² is selected from aminoalcohol groups represented by formulas (3) and (4) below: 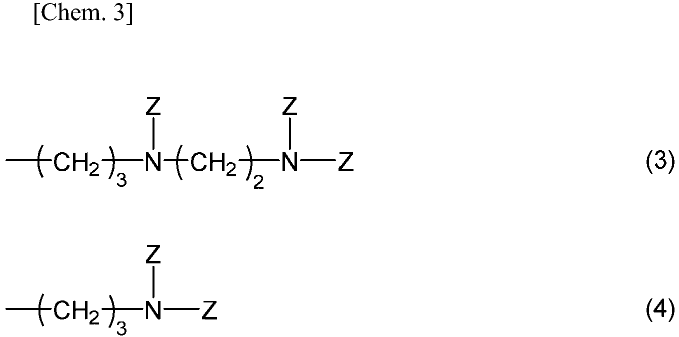 (wherein Z is the same as defined above).
3. The water-in-oil cosmetic according to 1 or 2, wherein in formula (1), b is an integer of 1 to 10, and a/b is 10 to 30.
4. The water-in-oil cosmetic according to any one of 1 to 3, wherein in formula (1), 99 to 100 mol% of the number of moles of all Zs in the molecule are -CH₂CH(OH)CH₃ or -CH(CH₃)CH₂OH.
5. The cosmetic according to any one of 1 to 4, wherein the ingredient (a) has a kinematic viscosity of 100 to 3,500 mm²/s.
6. The water-in-oil cosmetic according to any one of 1 to 5, wherein the ingredient (b) is one or more selected from sodium chloride and magnesium sulfate.
7. The water-in-oil cosmetic according to any one of 1 to 6, further including a water-soluble polyhydric alcohol (c).

### ADVANTAGEOUS EFFECTS OF INVENTION

The cosmetic of the present invention has a small emulsion particle size, gives a good feeling on use, and exhibits excellent viscosity stability over time even when having a low viscosity. The cosmetic can exhibit excellent viscosity stability over time even when the cosmetic contains an ingredient that is detrimental to emulsion viscosity stability, such as ethanol.

### DESCRIPTION OF EMBODIMENTS

The present invention will be described in detail below. In the present invention, the name of an ingredient is written as the name cited in *Kesho̅hin Hyo̅ji Meisho̅* [Japanese Cosmetic Labeling Names] or in the International Nomenclature of Cosmetic Ingredients (INCI). When the names from *Kesho̅hin Hyo̅ji Meisho̅* and the INCI are the same, the name from *Kesho̅hin Hyo̅ji Meisho̅* or the INCI name is sometimes omitted.

### [Ingredients (a)]

The ingredient (a) in the present invention is an aminoalcohol-modified silicone. The aminoalcohol-modified silicones may be used singly, or two or more may be used in combination. Examples of the aminoalcohol-modified silicones include aminoalcohol-modified silicones represented by formula (1) below: 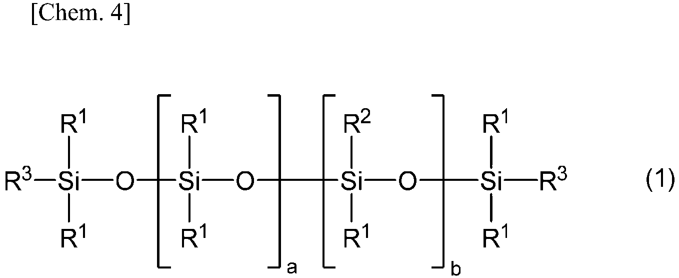 [R¹ independently at each occurrence is a monovalent hydrocarbon group selected from C1-C20 alkyl groups, C6-C15 aryl groups, and C7-C15 aralkyl groups; R² independently at each occurrence is a group represented by formula (2) below: 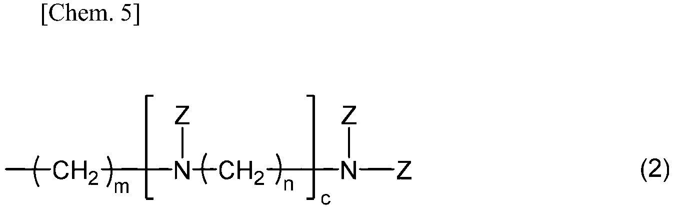 (Z independently at each occurrence is a group selected from hydrogen atom, -CH₂CH(OH)CH₃, and -CH(CH₃)CH₂OH; 50 mol% or more of the number of moles of all Zs in the molecule are groups selected from -CH₂CH(OH)CH₃ and -CH(CH₃)CH₂OH; m is an integer of 1 to 10; n is an integer of 1 to 5; and c is an integer of 0 to 4); R³ is R¹ or R²; a is 1 to 100; b is 0 to 10; and when b = 0, at least one of R³s is R²]. In the present invention, the order in which the siloxane units are bonded is not limited to that illustrated in the formula (the same applies hereinafter).

R¹ independently at each occurrence is a monovalent hydrocarbon group selected from C1-C20 alkyl groups, C6-C15 aryl groups, and C7-C15 aralkyl groups. Specific examples include alkyl groups, such as methyl group, ethyl group, propyl group, butyl group, and pentyl group; cycloalkyl groups, such as cyclopentyl group; and aryl groups, such as phenyl group and tolyl group. R¹ is preferably a methyl group or a phenyl group, and more preferably a methyl group.

R² independently at each occurrence is a group represented by formula (2) below: 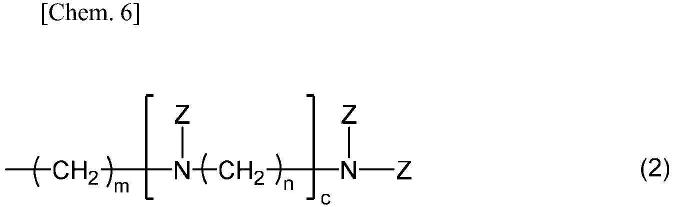 (Z independently at each occurrence is a group selected from hydrogen atom, -CH₂CH(OH)CH₃, and -CH(CH₃)CH₂OH; 50 mol% or more of the number of moles of all Zs in the molecule are groups selected from -CH₂CH(OH)CH₃ and -CH(CH₃)CH₂OH; m is an integer of 1 to 10; n is an integer of 1 to 5; and c is an integer of 0 to 4).

In formula (2), 50 mol% or more of the number of moles of all Zs in the molecule are groups selected from -CH₂CH(OH)CH₃ and -CH(CH₃)CH₂OH. This proportion is preferably 90 mol% or more, and more preferably 99 to 100 mol%. The letter m is an integer of 1 to 10, and preferably 1 to 5. The letter n is an integer of 1 to 5, and preferably 1 to 3. By virtue of the above proportion being 50 mol% or more, the compound is capable of allowing small particles to be emulsified and the over-time discoloration stemming from amino groups can be reduced.

R³ is R¹ or R² described above. The letter a is 1 to 100, preferably 10 to 80, and more preferably 20 to 60. If the letter a is greater than 100, the feeling on use is deteriorated. The letter b is 0 to 10, preferably 1 to 10, and more preferably 1 to 6. The letter c is an integer of 0 to 4, and more preferably 0 to 1. When b = 0, at least one of R³s is R². It is preferable that the letter b be an integer of 1 to 10, and a/b be 10 to 30, more preferably 10 to 20. In particular, formula (1) is preferably such that R² is a group selected from formulas (3) and (4) below: 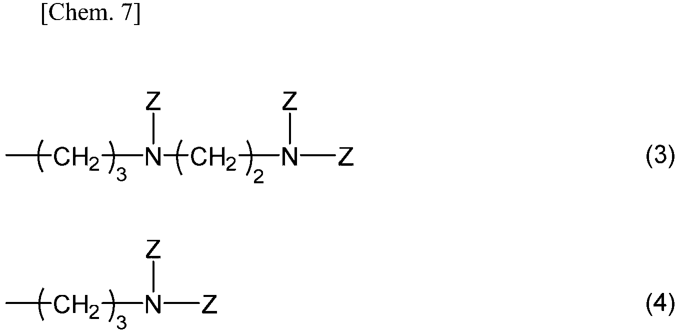 (wherein Z is the same as defined above). Furthermore, it is preferable that 99 mol% or more of the number of moles of all Zs in the molecule be groups selected from -CH₂CH(OH)CH₃ and -CH(CH₃)CH₂OH.

The kinematic viscosity of the ingredient (a) at 25°C is preferably 100 to 3,500 mm²/s, and more preferably 100 to 1,500 mm²/s. Controlling the kinematic viscosity to 3,500 mm²/s or less can further reduce the deterioration in the feeling on use of the emulsion due to the viscosity of the aminoalcohol-modified silicone. The kinematic viscosity is a value measured at 25°C using a Cannon-Fenske viscometer as described in JIS Z8803.

The amount in which the ingredient (a) is added is preferably 0.1 to 15 mass%, more preferably 0.2 to 10 mass%, still more preferably 0.5 to 8 mass%, and particularly preferably 0.5 to 5 mass% of the whole cosmetic. When the amount added is 0.1 mass% or more, the viscosity stability is enhanced. On the other hand, adding more than 15 mass% may result in a strong sticky feeling.

The production method is not particularly limited, and the ingredient (a) may be obtained by reacting an organopolysiloxane having an amino group with ethylene oxide, propylene oxide, or glycidol.

### [Ingredients (b)]

The ingredient (b) in the present invention is an inorganic salt. The inorganic salts may be used singly, or two or more may be used in combination. Examples of the inorganic salts include monovalent or divalent metal salts of inorganic acids, such as hydrochloric acid, sulfuric acid, carbonic acid, and nitric acid. Specific examples include sodium salts, potassium salts, magnesium salts, calcium salts, aluminum salts, zirconium salts, and zinc salts. In particular, sodium chloride (labeling name, INCI: Sodium Chloride) and magnesium sulfate (labeling name, INCI: Magnesium Sulfate) are preferable for their availability and high stabilizing effects.

The amount in which the ingredient (b) is added is 0.6 to 1.8 mass%, and preferably 0.8 to 1.5 mass% based on the whole cosmetic. If the amount added is less than 0.6 mass%, the emulsion fails to attain sufficient viscosity stability. If, on the other hand, the amount is larger than 1.8 mass%, the cosmetic may give a heavy feeling on use.

### [Ingredients (c)]

From the point of view of viscosity stability, it is preferable that the cosmetic of the present invention contain a water-soluble polyhydric alcohol (c). The water-soluble polyhydric alcohols are not particularly limited and may be any water-soluble polyhydric alcohols with two or more hydroxyl groups in the molecule that are usually added to cosmetics. Such water-soluble polyhydric alcohols may be used singly, or two or more may be used in combination. Specific examples include BG (labeling name, INCI: Butylene Glycol), PG (labeling name, INCI: Propylene Glycol), DPG (labeling name, INCI: Dipropylene Glycol), Pentylene Glycol (INCI), 1,10-Decanediol (INCI), Octanediol (INCI), 1,2-Hexanediol (INCI), Erythritol (INCI), Glycerin (INCI), Diglycerin (INCI), Polyethylene Glycol, Sorbitol (INCI), Maltose (INCI), Xylitol (INCI), and Glucose (INCI).

When the ingredient (c) is added, the amount thereof is preferably 3.5 to 70 mass%, more preferably 3.5 to 50 mass%, and particularly preferably 4 to 20 mass% based on the whole cosmetic.

### [Cosmetics]

The present invention is applicable to a variety of cosmetics, particularly preferably to cosmetics externally applied to the skin, such as skin care cosmetics, makeup cosmetics, antiperspirant cosmetics, and UV-protecting cosmetics, to cosmetics externally applied to the hair, such as hair care cosmetics, and to nail cosmetics.

Exemplary skin care cosmetics include skin lotions, milky lotions, creams, cleansing agents, packs, oil liquids, massage creams, cosmetic liquids, cosmetic oils, detergents, deodorants, hand creams, lip creams, and anti-wrinkle agents. Exemplary makeup cosmetics include makeup bases, foundations, concealers, cosmetic powders, cheek colors, eye colors, eye shadows, mascaras, eye liners, eyebrows, and lip cosmetics.

Exemplary antiperspirant cosmetics include antiperspirants of roll-on, cream, solution, and stick types. Exemplary UV-protecting cosmetics include sunscreen oils, sunscreen milky lotions, and sunscreen creams. Exemplary hair care cosmetics include shampoos, conditioners, treatments, and setting agents.

The cosmetic of the present invention is a water-in-oil cosmetic, and the type thereof may be any of W/O emulsion and O/W/O emulsion. The form of the cosmetic of the present invention may be selected from various forms including liquid, milky lotion, cream, solid, paste, gel, powder, pressed, multilayer, mousse, spray, stick, and pencil. The production method is not particularly limited and commonly known methods may be adopted.

The water-in-oil emulsion may be viscous or solid. When the emulsion is viscous, the viscosity is preferably 5,000 to 200,000 mPa·s, preferably more than 15,000 mPa·s and 150,000 mPa·s or less, or preferably 20,000 to 100,000 mPa·s. According to the present invention, it is possible to achieve stabilization of not only medium-viscosity and high-viscosity emulsions but also of low-viscosity emulsions. The viscosity is a value measured at 25°C using a Brookfield viscometer in accordance with the method described in JIS K 7117-1: 1999. For example, the Brookfield viscometer may be TVB-10 manufactured by Toki Sangyo Co., Ltd. A low-viscosity emulsion in the present invention is a sample having fluidity. Fluidity means that the sample having been stored in a vial at 25°C starts to flow within one minute after the vial is placed on its side.

The cosmetic of the present invention may contain various ingredients that are usually added to cosmetics without impairing the advantageous effects of the present invention. For example, such additional ingredients are (1) oils, (2) aqueous ingredients other than the ingredients (b) and (c), (3) surfactants, (4) powders, (5) compositions consisting of a crosslinked organopolysiloxane and an oil that is liquid at room temperature, (6) film-forming agents, (7) UV-absorbing/scattering agents, and (8) other additives. These may be each used singly, or two or more may be used in appropriate combination. The amounts of these ingredients may be selected appropriately in accordance with the type of cosmetic.

### (1) Oils

The oils may be volatile or nonvolatile and may be solid, semisolid, or liquid at room temperature (25°C). Examples include silicone oils, silicone waxes, natural animal and plant oils and fats, semi-synthetic oils and fats, hydrocarbon oils, higher alcohols, fatty acids, ester oils, fluorochemical oils, and UV absorbers.

### • Silicone oils

Examples of the silicone oils include Dimethicone (INCI); Trisiloxane (INCI); alkyl-modified silicones, such as Methyl Trimethicone (INCI), Ethyl Trisiloxane (INCI), Ethyl Methicone (INCI), and Hexyl Dimethicone (INCI); long-chain alkyl-modified silicones, such as Caprylyl Methicone (INCI); low- to high-viscosity, linear or branched organopolysiloxanes, such as Phenyl Trimethicone (INCI), Diphenyl Dimethicone (INCI), Diphenylsiloxyphenyl Trimethicone (INCI), Tetraphenyl Dimethyldisiloxane (INCI), and Methylhydrogenpolysiloxane; cyclic organopolysiloxanes, such as Cyclotetrasiloxane (INCI), Cyclopentasiloxane (INCI), and Cyclohexasiloxane (INCI); amino-modified organopolysiloxanes, such as Amodimethicone (INCI) and Aminopropyl Dimethicone (INCI); pyrrolidone-modified organopolysiloxanes, such as PCA Dimethicone (INCI); pyrrolidone carboxylic acid-modified organopolysiloxanes; silicone rubbers, such as gum-like dimethylpolysiloxanes having a high degree of polymerization, gum-like amino-modified organopolysiloxanes, and gum-like dimethylsiloxane-methylphenylsiloxane copolymers; as well as low-viscosity organopolysiloxane solutions of silicone gums and rubbers; amino acid-modified silicones; fluorine-modified silicones; silicone resins; and silicone resin solutions. Examples of commercially available silicone oils include KF-96L-1cs, KF-96L-1.5cs, KF-96L-2cs, KF-96A-6cs, KF-4422, KF-4418, TMF-1.5, KF-54, KF-54HV, KF-56A, and KF-995 manufactured by Shin-Etsu Chemical Co., Ltd.

### • Solid oil ingredients

When a solid cosmetic is desired in the present invention, it is preferable to add an oil ingredient that is solid at 25°C. The oil ingredient that is solid at 25°C is preferably one having a melting point of 40°C or above, more preferably 60 to 110°C. Examples include waxes, hydrocarbons, esters, higher alcohols, and higher fatty acids. The solid oil ingredients are not particularly limited and may be any raw materials usually added to cosmetics. Specific examples include plant waxes, such as carnauba wax (INCI: Copernicia Cerifera (Carnauba) Wax), sugar cane wax, candelilla wax (INCI: Euphorbia Cerifera (Candelilla) Wax), purified candelilla wax, rice wax, Japan wax, jojoba wax, kapok wax, rice bran wax, myrica cerifera fruit wax, shea butter, cacao butter, Japan wax (INCI: Rhus Succedanea Fruit Wax), montan wax (INCI: Montan Wax), and hydrogenated castor oil isostearate; animal waxes, such as beeswax, beef tallow, beef bone fat, lard (INCI: Lard), horse fat (INCI: Horse Fat), mutton tallow, lanolin (INCI: Lanolin), Ericerus pela wax, shellac wax, and sperm wax; semi-synthetic waxes, such as lanolin esters, lanolin fatty acid esters, and beeswax acid esters; hydrogenated oils, such as hydrogenated castor oil and hydrogenated coconut oil; hydrocarbon waxes, such as solid paraffin, polyethylene, ceresin, ozokerite, and microcrystalline wax; wax esters, such as synthetic beeswax; amino acid stearyl alcohols, such as dioctyldodecyl lauroylglutamate, dioctyldodecyl lauroylglutamate, and dioctyldodecyl lauroylglutamate; fatty acids, such as stearic acid and behenic acid; silicone waxes, such as acrylate silicone resins in the form of acrylate silicone graft or block copolymers (such as acrylate silicone graft copolymers: KP-561P and KP-562P manufactured by Shin-Etsu Chemical Co., Ltd.); and derivatives thereof. Any one, or two or more selected from the foregoing are preferable.

### • Natural animal and plant oils and semi-synthetic oils

Examples of the natural animal and plant oils and the semi-synthetic oils include natural plant oils, such as avocado oil (labeling name, INCI: Persea Gratissima (Avocado) Oil), linseed oil (labeling name, INCI: Linum Usitatissimum (Linseed) Seed Oil), almond oil (labeling name, INCI: Prunus Amygdalus Dulcis (Sweet Almond) Oil), perilla oil (labeling name), olive oil (labeling name, INCI: Olea Europaea (Olive) Fruit Oil), Torreya californica oil (labeling name, INCI: Torreya Californica (California Nutmeg) Oil), citronella grass oil (labeling name, INCI: Cymbopogon Nardus (Citronella) Oil), Torreya Nucifera seed oil (labeling name, INCI: Torreya Nucifera Seed Oil), apricot kernel oil (labeling name, INCI: Kyounin Yu), wheat germ oil (labeling name, INCI: Triticum Vulgare (Wheat) Germ Oil), sesame oil (labeling name, INCI: Sesamum Indicum (Sesame) Seed Oil), rice germ oil (labeling name, INCI: Oryza Sativa (Rice) Germ Oil), rice bran oil (labeling name, INCI: Oryza Sativa (Rice) Bran Oil), sasanqua oil (labeling name, INCI: Camellia Kissi Seed Oil), safflower oil (labeling name, INCI: Carthamus Tinctorius (Safflower) Seed Oil), soybean oil (labeling name, INCI: Glycine Soja (Soybean) Oil), tea seed oil (labeling name, INCI: Camellia Sinensis Seed Oil), camellia oil (labeling name, INCI: Camellia Japonica Seed Oil), evening primrose oil (labeling name, INCI: Oenothera Biennis (Evening Primrose) Oil), rapeseed oil (labeling name), germ oils, for example, corn germ oil (labeling name, INCI: Zea Mays (Corn) Germ Oil), persic oil (labeling name), palm oil (labeling name, INCI: Elaeis Guineensis (Palm) Oil), palm kernel oil (labeling name, INCI: Elaeis Guineensis (Palm) Kernel Oil), castor oil (labeling name, INCI: Ricinus Communis (Castor) Seed Oil), sunflower oil (labeling name, INCI: Helianthus Annuus (Sunflower) Seed Oil), grape seed oil (labeling name, INCI: Vitis Vinifera (Grape) Seed Oil), jojoba seed oil (labeling name, INCI: Simmondsia Chinensis (Jojoba) Seed Oil), macadamia seed oil (labeling name, INCI: Macadamia Ternifolia Seed Oil), meadowfoam oil (labeling name, INCI: Limnanthes Alba (Meadowfoam) Seed Oil), cotton seed oil (labeling name, INCI: Gossypium Herbaceum (Cotton) Seed Oil), coconut oil (labeling name, INCI: Cocos Nucifera (Coconut) Oil), and peanut oil (labeling name, INCI: Arachis Hypogaea (Peanut) Oil); natural animal oils, such as shark liver oil (labeling name, INCI: Shark Liver Oil), cod liver oil (labeling name, INCI: Cod Liver Oil), fish liver oil (labeling name, INCI: Fish Liver Oil), turtle oil (labeling name, INCI: Turtle Oil), mink oil (labeling name, INCI: Mink Oil), and egg yolk oil (labeling name, INCI: Egg Oil); and semi-synthetic oils and fats, such as hydrogenated coconut oil (labeling name, INCI: Hydrogenated Coconut Oil) and liquid lanolin (labeling name, INCI: Lanolin Oil).

### • Hydrocarbon oils

Examples of the hydrocarbon oils include linear or branched hydrocarbon oils.
The hydrocarbon oils may be volatile or nonvolatile. Specific examples include alkanes, such as Olefin Oligomers (INCI), isoparaffins, for example, (C13, 14) Isoparaffins (INCI), Isododecane (INCI), Undecane (INCI), Dodecane (INCI), Isohexadecane (INCI), hydrogenated polyisobutene (labeling name, INCI: Hydrogenated Polyisobutene), Squalane (INCI), Mineral Oil (INCI), Coconut Alkanes (INCI), and (C13-15) Alkanes (INCI).

### • Higher alcohols

Examples of the higher alcohols include linear saturated alcohols having 6 or more carbon atoms, such as Lauryl Alcohol (INCI), Hexyldecanol (INCI), Oleyl Alcohol (INCI), Isostearyl Alcohol (INCI), Octyldodecanol (INCI), Decyltetradecanol (INCI), Myristyl Alcohol (INCI), Cetyl Alcohol (INCI), Stearyl Alcohol (INCI), and Behenyl Alcohol (INCI), as well as Batyl Alcohol (INCI). Examples further include sterols, such as Cholesterol (INCI), sitosterol (labeling name, INCI: Beta-Sitosterol)), Phytosterols (INCI), and Lanosterol (INCI).

### • Ester oils

Examples of the ester oils include diisobutyl adipate (labeling name, INCI: Diisobutyl Adipate), dihexyldecyl adipate (labeling name), diheptylundecyl adipate (labeling name, INCI: Diheptylundecyl Adipate), alkylglycol monoisostearates, such as isostearyl isostearate (labeling name, INCI: Isostearyl Isostearate), isocetyl isostearate (labeling name, INCI: Isocetyl Isostearate), trimethylolpropane triisostearate (labeling name, INCI: Trimethylolpropane Triisostearate), glycol diethylhexanoate (labeling name, INCI: Glycol Diethylhexanoate), cetyl ethylhexanoate (labeling name, INCI: Cetyl Ethylhexanoate), trimethylolpropane triethylhexanoate (labeling name, INCI: Trimethylolpropane Triethylhexanoate), pentaerythrityl tetraethylhexanoate (labeling name, INCI: Pentaerythrityl Tetraethylhexanoate), octyldodecyl esters, such as octyldodecyl stearoyloxystearate (labeling name, INCI: Octyldodecyl Stearoyl Stearate), oleyl oleate (labeling name, INCI: Oleyl Oleate), octyldodecyl oleate (labeling name, INCI: Octyldodecyl Oleate), decyl oleate (labeling name, INCI: Decyl Oleate), neopentyl glycol dioctanoate (labeling name, INCI: Neopentyl Glycol Diethylhexanoate), neopentyl glycol dicaprate (labeling name, INCI: Neopentyl Glycol Dicaprate), diisostearyl malate (labeling name, INCI: Diisostearyl Malate), triethyl citrate (labeling name, INCI: Triethyl Citrate), diethylhexyl succinate (labeling name, INCI: Diethylhexyl Succinate), amyl acetate (labeling name, INCI: Amyl Acetate), ethyl acetate (labeling name, INCI: Ethyl Acetate), butyl acetate (labeling name, INCI: Butyl Acetate), isocetyl stearate (labeling name, INCI: Isocetyl Stearate), butyl stearate (labeling name, INCI: Butyl Stearate), diisopropyl sebacate (labeling name, INCI: Diisopropyl Sebacate), diethylhexyl sebacate (labeling name, INCI: Diethylhexyl Sebacate), cetyl lactate (labeling name, INCI: Cetyl Lactate), myristyl lactate (labeling name, INCI: Myristyl Lactate), isononyl isononanoate (labeling name, INCI: Isononyl Isononanoate), isotridecyl isononanoate (labeling name, INCI: Isotridecyl Isononanoate), palmitates, such as isopropyl palmitate (labeling name, INCI: Isopropyl Palmitate), ethylhexyl palmitate (labeling name, INCI: Ethylhexyl Isopalmitate), and hexyldecyl palmitate (labeling name, INCI: Isocetyl Palmitate, Hexyldecyl Palmitate), cholesteryl (labeling name, INCI: Cholesteryl Hydroxystearate), myristates, such as isopropyl myristate (labeling name, INCI: Isopropyl Myristate), octyldodecyl myristate (labeling name, INCI: Octyldodecyl Myristate), and myristyl myristate (labeling name, INCI: Myristyl Myristate), ethylhexyl laurate (labeling name, INCI: Ethylhexyl Laurate), hexyl laurate (labeling name, INCI: Hexyl Laurate), dioctyldodecyl lauroyl glutamate (labeling name, INCI: Dioctyldodecyl Lauroyl Glutamate), isopropyl lauroyl sarcosinate (labeling name, INCI: Isopropyl Lauroyl Sarcosinate), and coco-alkyl caprylate/caprate (labeling name, INCI: Coco-Caprylate/Caprate).

Among the ester oils, exemplary glyceride oils include Triethylhexanoin (INCI), glyceryl tri(caprylate/caprate) (labeling name, INCI: Caprylic/Capric Triglyceride), Cocoglyceryl (INCI), triglyceryl caprylate/caprate/succinate (labeling name, INCI: Caprylic/Capric/Succinic Triglyceride), and caprylic/capric glycerides (labeling name, INCI: Caprylic/Capric Glycerides).

### • Fluorochemical oils

Examples of the fluorochemical oils include Perfluorodecalin (INCI), Perfluorononyl Dimethicone (INCI), and Perfluoromethylcyclopentane (INCI).

### • UV absorbers

Examples of the UV absorbers include oxybenzone-1 (labeling name, INCI: Benzophenone-1), oxybenzone-2 (labeling name, INCI: Benzophenone-2), oxybenzone-3 (labeling name, INCI: Benzophenone-3), oxybenzone-4 (labeling name, INCI: Benzophenone-4), oxybenzone-5 (labeling name, INCI: Benzophenone-5), oxybenzone-6 (labeling name, INCI: Benzophenone-6), oxybenzone-9 (labeling name, INCI: Benzophenone-9), Homosalate (INCI), Octocrylene (INCI), t-butyl methoxydibenzoylmethane (labeling name, INCI: Butyl Methoxydibenzoylmethane), ethylhexyl salicylate (labeling name, INCI: Ethylhexyl Salicylate), diethylaminohydroxybenzoyl hexyl benzoate (labeling name, INCI: Diethylamino Hydroxybenzoyl Hexyl Benzoate), Polysilicone-15 (INCI), dimethoxybenzylidene dioxoimidazolidine octyl propionate (labeling name, INCI: Ethylhexyl Dimethoxybenzylidene Dioxoimidazolidine Propionate), terephthalylidene dicamphor sulfonic acid (labeling name, INCI: Terephthalylidene Dicamphor Sulfonic Acid), Ethylhexyl Triazone (INCI), methylbis(trimethylsiloxy)silyl isopentyl trimethoxycinnamate (labeling name, INCI: Isopentyl Trimethoxycinnamate Trisiloxane), Drometrizole Trisiloxane (INCI), dimethyl PABA ethylhexyl (labeling name, INCI: Ethylhexyl Dimethyl PABA), isopropyl para-methoxycinnamate (labeling name, INCI: Isopropyl Methoxycinnamate), ethylhexyl methoxycinnamate (labeling name, INCI: Ethylhexyl Methoxycinnamate), Bis-Ethylhexyloxyphenol Methoxyphenyltriazine (INCI), phenylbenzimidazole sulfonic acid (labeling name, INCI: Phenylbenzimidazole Sulfonic Acid), Methylene Bis-Benzotriazolyl Tetramethylbutylphenol (INCI), glyceryl ethylhexanoate dimethoxycinnamate (labeling name, INCI: Glyceryl Ethylhexanoate Dimethoxycinnamate), Glyceryl PABA (INCI), methyl diisopropylcinnamate (labeling name, INCI: Diisopropyl Methyl Cinnamate), Cinoxate (INCI), and ethylhexyl dimethoxybenzylidene dioxoimidazolidine propionate (labeling name, INCI: Ethylhexyl Dimethoxybenzylidene Dioxoimidazolidine Propionate). Furthermore, a UVA absorber (such as, for example, Diethylamino Hydroxybenzoyl Hexyl Benzoate) and a UVB absorber (such as, for example, Ethylhexyl Methoxycinnamate) may be used in combination, or absorbers belonging to the same type may be combined appropriately.

The amount in which the oil is added is preferably 8 to 60 mass%, and more preferably 12 to 35 mass% based on the whole cosmetic.

### (2) Aqueous ingredients

The aqueous ingredients are not particularly limited as long as they are aqueous ingredients that are usually added to cosmetics. Specific examples include water; lower alcohols preferably having 2 to 5 carbon atoms, such as ethanol (labeling name, INCI: Alcohol) and isopropanol (labeling name, INCI: Isopropyl Alcohol); and moisturizers, such as Glyceryl Glucoside (INCI), Betaine (INCI), sodium chondroitin sulfate (labeling name, INCI: Sodium Chondroitin Sulfate), PCA-Na (labeling name, INCI: Sodium PCA), Methyl Gluceth-10 (INCI), Methyl Gluceth-20 (INCI), hyaluronic acid, egg yolk lecithin, soybean lecithin, phosphatidylcholine, phosphatidylethanolamine, phosphatidylserine, phosphatidylglycerol, phosphatidylinositol, and sphingophospholipid. The amount in which the aqueous ingredient is added may be controlled appropriately and may be the balance left over after the deduction of all the ingredients constituting the whole cosmetic.

Ethanol as an aqueous ingredient generally deteriorates the emulsion viscosity stability of a water-in-oil emulsion cosmetic. However, the addition of the ingredients (a) and (b) and optionally of the ingredient (c) of the present invention can stabilize such a cosmetic. Ethanol is useful to adjust the feeling on use and to solubilize various hardly soluble ingredients. Thus, the availability of ethanol expands the variety of cosmetics that can be prepared. The amount in which ethanol is added is preferably 0.1 to 20 mass%, and more preferably 0.5 to 15 mass% of the whole cosmetic, and is particularly preferably 1 to 10 mass% in terms of the feeling on use.

### (3) Surfactants

The surfactants may be nonionic, anionic, cationic, or ampholytic. The surfactants are not particularly limited and any surfactants usually used in cosmetics may be used. Among such surfactants, one, or two or more selected from non-crosslinked silicone surfactants and crosslinked silicone surfactants are preferable because stable cosmetics can be obtained. For any types of surfactants, the amount is preferably 0.1 to 20 mass% of the whole of the cosmetic. An amount of 0.1 mass% or more ensures that the dispersing and emulsifying functions are fully achieved whereas an amount of 20 mass% or less advantageously eliminates the risk that the cosmetic gives a sticky feeling on use. The HLB of the surfactants is not limited but is preferably 2 to 14.5 in order to maintain the water resistance of the cosmetic.

The non-crosslinked silicone surfactants are linear or branched silicones in which some methyl groups on the backbone are substituted with hydrophilic groups, such as polyethylene glycol or polyglycerin. Specifically, preferred non-crosslinked silicone surfactants are linear or branched polyoxyethylene-modified organopolysiloxanes, linear or branched polyoxyethylene polyoxypropylene-modified organopolysiloxanes, linear or branched polyoxyethylene/alkyl-co-modified organopolysiloxanes, linear or branched polyoxyethylene polyoxypropylene/alkyl-co-modified organopolysiloxanes, linear or branched polyglycerin-modified organopolysiloxanes, linear or branched polyglycerin/alkyl-co-modified organopolysiloxanes, and linear or branched pyrrolidone-modified organopolysiloxanes. Examples include PEG-11 Methyl Ether Dimethicone (INCI), PEG/PPG-20/22 Butyl Ether Dimethicone (INCI), PEG-3 Dimethicone (INCI), PEG-10 Dimethicone (INCI), PEG-9 Polydimethylsiloxyethyl Dimethicone (INCI), Lauryl PEG-9 Polydimethylsiloxyethyl Dimethicone (INCI), Cetyl PEG/PPG-10/1 Dimethicone (INCI), Polyglyceryl-3 Disiloxane Dimethicone (INCI), Polyglyceryl-3 Polydimethylsiloxyethyl Dimethicone (INCI), Lauryl Polyglyceryl-3 Polydimethylsiloxyethyl Dimethicone (INCI), and Bis-Butyl Dimethicone Polyglyceryl-3 (INCI).

Examples of commercially available products include KF-6011, KF-6011P, KF-6012, KF-6015, KF-6017, KF-6043, KF-6028, KF-6038, KF-6048, KF-6100, KF-6104, KF-6106, KF-6105, and KF-6115 manufactured by Shin-Etsu Chemical Co., Ltd.

Examples of the crosslinked silicone surfactants include crosslinked polyether-modified silicones, such as (Dimethicone/(PEG-10/15)) Crosspolymer (INCI), (PEG-15/Lauryl Dimethicone) Crosspolymer (INCI), (PEG-10/Lauryl Dimethicone) Crosspolymer (INCI), and (PEG-15/Lauryl Polydimethylsiloxyethyl Dimethicone) Crosspolymer (INCI); and crosslinked polyglycerin-modified silicones, such as (Dimethicone/Polyglycerin-3) Crosspolymer (INCI), (Lauryl Dimethicone/Polyglycerin-3) Crosspolymer (INCI), and (Polyglycerin-3/Lauryl Polydimethylsiloxyethyl Dimethicone) Crosspolymer (INCI). When the crosslinked silicone surfactant is used as a composition consisting of the crosslinked silicone surfactant and an oil that is liquid at room temperature, it is preferable that the crosslinked silicone surfactant be swollen by containing its own weight or more of the liquid oil.

The liquid oil may be a liquid silicone oil, a hydrocarbon oil, an ester oil, a natural animal or plant oil, a semi-synthetic oil, or a fluorochemical oil belonging to the optional oil ingredients (1), with examples including Cyclopentasiloxane (INCI), Dimethicone (INCI), Mineral Oil (INCI), Isododecane (INCI), Isohexadecane (INCI), Triethylhexanoin (INCI), isotridecyl isononanoate (labeling name, INCI: Isotridecyl Isononanoate), and Squalane (INCI).

Commercially available crosslinked silicone surfactants swollen by containing a liquid oil include, for example, KSG-210, KSG-240, KSG-270, KSG-310, KSG-320, KSG-330, KSG-340, KSG-320Z, KSG-350Z, KSG-710, KSG-810, KSG-820, KSG-830, KSG-840, KSG-820Z, and KSG-850Z manufactured by Shin-Etsu Chemical Co., Ltd. In the present invention, the cosmetic may be free of polyglycerin-modified silicone surfactants, such as partially crosslinked polyglycerin-modified silicones, linear or branched polyglycerin-modified organopolysiloxanes, and linear or branched polyglycerin/alkyl-co-modified organopolysiloxanes.

### (4) Powders

Powders may be added. Examples of the powders include coloring pigments, inorganic powders, metal powders, organic powders, and inorganic-organic composite powders. These powders are described in detail below.

### • Coloring pigments

The coloring pigments are not particularly limited and may be any pigments commonly used in cosmetics for the purpose of coloring. Examples include red iron oxide (labeling name, INCI: Iron Oxides), yellow iron oxide (labeling name, INCI: Iron Oxides), white titanium dioxide (labeling name, INCI: Titanium Dioxide), black iron oxide (labeling name, INCI: Iron Oxides), ultramarine (labeling name, INCI: Ultramarines), Prussian blue (labeling name, INCI: Ferric Ferrocyanide, Ferric Ammonium Ferrocyanide), manganese violet (labeling name, INCI: Manganese Violet), cobalt titanate (labeling name, INCI: Cobalt Titanium Dioxide), chromium hydroxide (labeling name, INCI: Chromium Hydroxide Green), chromium oxide (labeling name, INCI: Chromium Oxide Greens), Al/cobalt oxide (labeling name, INCI: Cobalt Aluminum Oxide), fired titanium/titanium dioxide (labeling name, INCI: Titanium/Titanium Dioxide), Li/cobalt titanate (labeling name, INCI: Lithium Cobalt Titanate), sintered iron oxide/titanium dioxide (labeling name), composites doped with a different metal, such as iron oxide-doped titanium dioxide (labeling name, INCI: Iron Oxides, Titanium Dioxide), titanium nitride (labeling name, INCI: Titanium Nitride), ferrous hydroxide (labeling name, INCI: Iron Hydroxide), inorganic brown pigments, such as γ-iron oxide, inorganic yellow pigments, such as loess, and colored pigments, such as lake form of tar dyes and lake form of natural dyes. Any of the foregoing may be used.

The pigments may take any shape, with examples including spherical, generally spherical, bar, spindle, petal, strip, and irregular shapes. Their geometry is not particularly limited as long as the pigments can impart a color to the cosmetics.

### • Inorganic powders

Examples of the inorganic powders include microparticles of zirconium oxide (labeling name, INCI: Zirconium Dioxide), zinc oxide (labeling name, INCI: Zinc Oxide), cerium oxide (labeling name, INCI: Cerium Oxide), Mg oxide (labeling name, INCI: Magnesium Oxide), Ba sulfate (labeling name, INCI: Barium Sulfate), calcium sulfate (labeling name, INCI: Calcium Sulfate), Mg sulfate (labeling name, INCI: Magnesium Sulfate), Ca carbonate (labeling name, INCI: Calcium Carbonate), Mg carbonate (labeling name, INCI: Magnesium Carbonate), Talc (INCI), Mica (INCI), Kaolin (INCI), synthetic fluorphlogopite (labeling name, INCI: Synthetic Fluorphlogopite), synthetic ferrous phlogopite (labeling name), black mica (labeling name, INCI: Biotite), K silicate (labeling name, INCI: Potassium Silicate), Silica (INCI), Al silicate (labeling name, INCI: Aluminum Silicate), Mg silicate (labeling name, INCI: Magnesium Silicate), Al/Mg silicate (labeling name, INCI: Magnesium Aluminum Silicate), Ca silicate (labeling name, INCI: Calcium Silicate), Al/Ca/Na silicate (labeling name, INCI: Aluminum Calcium Sodium Silicate), Li/Mg/Na silicate (labeling name, INCI: Lithium Magnesium Sodium Silicate), Na/Mg silicate (labeling name, INCI: Sodium Magnesium Silicate), Ca/Al borosilicate (labeling name, INCI: Calcium Aluminum Borosilicate), Ca/Na borosilicate (labeling name, INCI: Calcium Sodium Borosilicate), Hydroxyapatite (INCI), Bentonite (INCI), Montmorillonite (INCI), Hectorite (INCI), Zeolite (INCI), Alumina (INCI), Al hydroxide (labeling name, INCI: Aluminum Hydroxide), boron nitride (labeling name, INCI: Boron Nitride), and glass (labeling name, INCI: Glass).

Furthermore, examples of the inorganic coloring pearly pigments include pearly agents, such as Mica (INCI) coated with titanium dioxide (labeling name, INCI: Titanium Dioxide) and synthetic fluorphlogopite (labeling name, INCI: Synthetic Fluorphlogopite) coated with titanium dioxide (labeling name, INCI: Titanium Dioxide); and pearly pigments, such as bismuth oxychloride (labeling name, INCI: Bismuth Oxychloride), bismuth oxychloride (labeling name, INCI: Bismuth Oxychloride) coated with titanium dioxide (labeling name, INCI: Titanium Dioxide), Talc (INCI) coated with titanium dioxide (labeling name, INCI: Titanium Dioxide), fish scale flakes (labeling name), and coloring mica coated with titanium dioxide (labeling name, INCI: Titanium Dioxide). These may be untreated or may have undergone well-known surface treatment generally used for cosmetic products.

### • Metal powders

Examples of the metal powders include metal microparticles of Al (labeling name, INCI: Aluminum, Aluminum Powder), copper (labeling name, INCI: Copper Powder), silver (labeling name, INCI: Silver Powder), and gold (labeling name, INCI: Gold).

### • Organic powders

Examples of the organic powders include powders of silicones, polyamides, polyacrylic acids/acrylates, polyesters, Polyethylene (INCI), Polypropylene (INCI), Polystyrene (INCI), styrene/acrylic acid copolymers, divinylbenzene/styrene copolymers, polyurethanes, vinyl resins, urea resins, melamine resins, benzoguanamine, polymethyl benzoguanamine, tetrafluoroethylene, polymethyl methacrylate, Cellulose (INCI), Silk (INCI), nylon (labeling name), phenolic resins, epoxy resins, and polycarbonates.

In particular, examples of the silicones include silicone resin particles; Polymethylsilsesquioxane (INCI), silicone rubber powders, silicone resin-coated silicone rubber powders; vinyl dimethicone/methicone silsesquioxane crosspolymer (labeling name, INCI: Vinyl Dimethicone/Methicone Silsesquioxane Crosspolymer), diphenyl dimethicone/vinyldiphenyl dimethicone/silsesquioxane crosspolymer (labeling name, INCI: Diphenyl Dimethicone/Vinyl Diphenyl Dimethicone/Silsesquioxane Crosspolymer), Polysilicone-1 Crosspolymer (INCI), and Polysilicone-22 (INCI).

Examples of commercially available silicone powders include KMP-590, KMP-591, KMP-592, KMP-597, KMP-598, KSP-100, KSP-101, KSP-102, KSP-105, KSP-300, KSP-411, KSP-441, KM-9729, and KM-440 manufactured by Shin-Etsu Chemical Co., Ltd.

Examples further include powders of metal soaps, specifically, zinc stearate (labeling name, INCI: Zinc Stearate), Al stearate (labeling name, INCI: Aluminum Stearate), Ca stearate (labeling name, INCI: Calcium Stearate), Mg stearate (labeling name, INCI: Magnesium Stearate), zinc myristate (labeling name, INCI: Zinc Myristate), Mg myristate (labeling name, INCI: Magnesium Myristate), zinc/Na cetylphosphate (labeling name, INCI: Sodium Zinc Cetyl Phosphate), and K cetylphosphate (labeling name, INCI: Potassium Cetyl Phosphate).

Examples further include organic colorants, specifically, tar dyes, such as Red #3, Red #104(1) (labeling name, INCI: Red 28, Red 28 Lake), Red #106, Red #201 (labeling name, INCI: Red 6), Red #202 (labeling name, INCI: Red 7), Red #204, Red #205, Red #220 (labeling name, INCI: Red 34), Red #226 (labeling name, INCI: Red 30), Red #227 (labeling name, INCI: Red 33, Red 33 Lake), Red #228 (labeling name, INCI: Red 36), Red #230(1) (labeling name, INCI: Red 22, Red 22 Lake), Red #230(2) (labeling name), Red #401 (labeling name), Red #505 (labeling name), Yellow #4 (labeling name, INCI: Yellow 5), Yellow #5 (labeling name, INCI: Yellow 6, Yellow 6 Lake), Yellow #202(1) (labeling name, INCI: Yellow 8), Yellow #203 (labeling name, INCI: Yellow 10, Yellow 10 Lake), Yellow #204 (labeling name, INCI: Yellow 11), Yellow #401, Blue #1 (labeling name, INCI: Blue 1, Blue 1 Lake), Blue #2, Blue #201, Blue #205 (labeling name, INCI: Blue 4), Blue #404 (labeling name), Green #3 (labeling name, INCI: Green 3, Green 3 Lake), Green #201 (labeling name, INCI: Green 5), Green #202 (labeling name, INCI: Green 6), Green #204 (labeling name, INCI: Green 8), Green #205 (labeling name), Orange #201 (labeling name, INCI: Orange 5), Orange #203 (labeling name, INCI: Pigment Orange 5), Orange #204 (labeling name), Orange #205 (labeling name, INCI: Orange 4, Orange 4 Lake), Orange #206 (labeling name, INCI: Orange 10), and Orange #207 (labeling name, INCI: Orange 11); and natural dyes, such as Cochineal (INCI), laccaic acid (labeling name, INCI: Laccaic Acid), safflower red (labeling name, INCI: Carthamus Tinctorius (Safflower) Flower Extract), red root extract (labeling name, INCI: Lithospermum Officinale Root Extract), gardenia yellow (labeling name), and gardenia blue (labeling name, INCI: Hydrolyzed Gardenia Florida Extract).

### • Inorganic/organic composite powders

Examples of the inorganic/organic composite powders include composite powders obtained by coating the surface of an inorganic powder with an organic powder by any well-known method.

The powders described hereinabove may be surface-treated. From the point of view of the water resistance of the cosmetic, the surface treatment agent is preferably one that can impart hydrophobicity. The hydrophobicity-imparting agent is not particularly limited, and examples thereof include silicone treatment agents, waxes, paraffins, organic fluorine compounds, such as perfluoroalkyl phosphates, surfactants, amino acids, such as N-acylglutamic acid, and metal soaps, such as aluminum stearate and magnesium myristate. Silicone treatment agents are preferable. Examples thereof include silanes or silylating agents, such as Triethoxycaprylylsilane (INCI), Dimethicone (INCI), Methicone (INCI), Hydrogen Dimethicone (INCI), Triethoxysilylethyl Polydimethylsiloxyethyl Dimethicone (INCI), Triethoxysilylethyl Polydimethylsiloxyethylhexyl Dimethicone (INCI), and acrylates/tridecyl acrylate/triethoxysilylpropyl methacrylate/dimethicone methacrylate copolymer (labeling name, INCI: Acrylates/Tridecyl Acrylate/Triethoxysilylpropyl Methacrylate/Dimethicone Methacrylate Copolymer). Specific examples of the silicone treatment agents include AES-3083, KF-99P, KF-9901, KF-9908, KF-9909, KP-574, and KP-541 manufactured by Shin-Etsu Chemical Co., Ltd. The surface hydrophobizing agents may be used singly, or two or more may be used in combination. Specific examples of the surface-treated coloring pigments include KTP-09 series, in particular, KTP-09W, 09R, 09Y, and 09B manufactured by Shin-Etsu Chemical Co., Ltd.

### (5) Compositions consisting of a crosslinked organopolysiloxane and an oil that is liquid at room temperature

In the composition consisting of a crosslinked organopolysiloxane and an oil that is liquid at room temperature, the crosslinked organopolysiloxane is preferably swollen by containing its own weight or more of the liquid oil. The liquid oil may be a liquid silicone oil, a hydrocarbon oil, an ester oil, a natural animal or plant oil, a semi-synthetic oil, or a fluorochemical oil. Examples include Cyclopentasiloxane (INCI), Dimethicone (INCI), Mineral Oil (INCI), Isododecane (INCI), Isohexadecane (INCI), Triethylhexanoin (INCI), isotridecyl isononanoate (labeling name, INCI: Isotridecyl Isononanoate), and Squalane (INCI).

Unlike the crosslinked silicone surfactants belonging to the ingredients (3), the ingredients (5) are compounds that do not have a polyether or polyglycerin structure in the molecular structure. Specific examples include Dimethicone/Vinyl Dimethicone Crosspolymer (INCI), Dimethicone/Phenyl Vinyl Dimethicone Crosspolymer (INCI), Vinyl Dimethicone/Lauryl Dimethicone Crosspolymer (INCI), and Lauryl Polydimethylsiloxyethyl Dimethicone/Bis-Vinyl Dimethicone Crosspolymer (INCI). Examples of the commercial compositions consisting of a crosslinked organopolysiloxane and an oil that is liquid at room temperature include KSG-15, KSG-1510, KSG-16, KSG-1610, KSG-19, KSG-016F, KSG-18A, KSG-41A, KSG-42A, KSG-43, KSG-44, KSG-042Z, KSG-045Z, and KSG-048Z manufactured by Shin-Etsu Chemical Co., Ltd.

### (6) Film-forming agents

The film-forming agent is added mainly for the purpose of further extending the durability of the effects of the cosmetic. The film-forming agents are not particularly limited. Silicone compositions are preferable from the point of view of imparting water repellency. Specifically, for example, use may be made of trimethylsiloxysilicic acid, acrylic-silicone film formers, silicone-modified norbornene, silicone-modified pullulan, and silicone-modified polyvinyl alcohol. Examples of the film-forming agents in the form of silicone compositions include trimethylsiloxysilicic acid (labeling name, INCI: Trimethylsiloxysilicate), Acrylates/Dimethicone Copolymer (INCI), Norbornene/Tris(Trimethylsiloxy)Silylnorbornene Copolymer (INCI), and pullulan tri(trimethylsiloxy)silylpropyl carbamide (labeling name, INCI: Trimethylsiloxysilylcarbamoyl Pullulan). The film-forming agent may be previously dissolved into an oil that is liquid at room temperature before being added to the cosmetic. The liquid oil may be a liquid silicone oil, a hydrocarbon oil, an ester oil, a natural animal or plant oil, a semi-synthetic oil, or a fluorochemical oil. Specific examples of commercially available silicone film-forming agents include KF-7312J, KP-545, KP-549, KP-543, NBN-30-ID, TSPL-30-ID, and TSPL-30-D5 manufactured by Shin-Etsu Chemical Co., Ltd.

### (7) UV-absorbing/scattering agents

Examples of the UV-absorbing/scattering agents include particles that absorb and scatter ultraviolet rays, such as titanium dioxide microparticles, iron-containing titanium dioxide microparticles, zinc oxide microparticles, cerium oxide microparticles, and composites thereof. These UV-absorbing/scattering particles may be used as a dispersion in an oil. The oil may be a liquid silicone oil, a hydrocarbon oil, an ester oil, a natural animal or plant oil, a semi-synthetic oil, or a fluorochemical oil. Specific examples of the oil dispersions of the UV-absorbing/scattering particles include SPD series (product name), in particular, SPD-T5, T5L, Z5, Z5L, T6, Z6, T7, and Z7L manufactured by Shin-Etsu Chemical Co., Ltd.

### (8) Other additives

Other additives include oil-soluble gelling agents, preservatives, antibacterial agents, antiperspirants, fragrances, salts, antioxidants, acidity regulators, chelating agents, refreshing agents, anti-inflammatory agents, skin modifying ingredients (such as brightening or whitening agents, cell activators, anti-skin-roughening agents, blood flow enhancing agents, skin astringents, and antiseborrheic agents), vitamins, amino acids, nucleic acids, hormones, and clathrate compounds.

### • Oil-soluble gelling agents

Exemplary oil-soluble gelling agents include metal soaps, such as aluminum stearate, magnesium stearate, and zinc myristate; amino acid derivatives, such as lauroyl glutamic acid (labeling name, INCI: Lauroyl Glutamic Acid) and α,γ-di-n-butylamine; dextrin fatty acid esters, such as dextrin palmitate (labeling name, INCI: Dextrin Palmitate), dextrin isostearate (labeling name, INCI: Dextrin Isostearate), dextrin myristate (labeling name, INCI: Dextrin Myristate), inulin stearate (labeling name, INCI: Stearoyl Inulin), and dextrin palmitate/ethylhexanoate (labeling name, INCI: Dextrin Palmitate/Ethylhexanoate); sucrose fatty acid esters, such as sucrose palmitate and sucrose stearate; fructooligosaccharide fatty acid esters, such as fructooligosaccharide stearate and fructooligosaccharide 2-ethylhexanoate; benzylidene derivatives of sorbitol, such as monobenzylidene sorbitol and dibenzylidene sorbitol; organo-modified clay minerals of hectorite, such as Disteardimonium Hectorite (INCI) and Stearalkonium Hectorite (INCI); and Stearalkonium Bentonite (INCI).

### • Preservatives and antibacterial agents

Examples of the preservatives and the antibacterial agents include alkyl p-hydroxybenzoates, benzoic acid, sodium benzoate, sorbic acid, potassium sorbate, phenoxyethanol, imidazolidinyl urea, salicylic acid, isopropyl methyl phenol, phenol, p-chloro-m-cresol, hexachlorophene, benzalkonium chloride, chlorohexidine chloride, trichlorocarbanilide, iodopropynyl butylcarbamate, polylysine, photosensitizers, silver, and plant extracts.

### • Antiperspirants

Examples of the antiperspirants include aluminum halohydrates, such as aluminum chlorohydrate, aluminum halides, such as aluminum chloride, aluminum allantoinate, tannic acid, persimmon tannin, aluminum potassium sulfate, zinc oxide, zinc p-phenolsulfonate, calcined alum, aluminum zirconium tetrachlorohydrate, and aluminum zirconium trichlorohydrex glycine. In particular, preferred ingredients that exert a higher effect include aluminum halohydrates, aluminum halides, and complexes or mixtures thereof with zirconium oxyhalides and zirconium hydroxyhalides (for example, aluminum zirconium tetrachlorohydrate and aluminum zirconium trichlorohydrex glycine).

### • Fragrances

The fragrances include natural fragrances and synthetic fragrances. Examples of the natural fragrances include plant fragrances separated from flowers, leaves, stems, and pericarps; and animal fragrances, such as musk and civet. Examples of the synthetic fragrances include hydrocarbons, such as monoterpene; alcohols, such as aliphatic alcohols and aromatic alcohols; aldehydes, such as terpene aldehydes and aromatic aldehydes; ketones, such as alicyclic ketones; esters, such as terpene esters; lactones; phenols; oxides; nitrogen-containing compounds; and acetals.

### • Salts other than the ingredients (b)

Examples of the salts include organic acid salts, amine salts, and amino acid salts. Exemplary organic acid salts include salts of organic acids, such as acetic acid, dehydroacetic acid, citric acid, malic acid, succinic acid, ascorbic acid, and stearic acid. Exemplary amine salts and amino acid salts include salts of amines, such as triethanol amine, and salts of amino acids, such as glutamic acid. Also useful are salts of hyaluronic acid, chondroitin sulfate, aluminum zirconium glycine complex, and acid-alkali neutral salts used in cosmetic formulations.

### • Antioxidants

Examples of the antioxidants include, but are not particularly limited to, carotenoid, ascorbic acid and salts thereof, ascorbyl stearate, tocopherol, tocopherol acetate, tocopherol, p-t-butylphenol, butyl hydroxyanisole, dibutyl hydroxytoluene, phytic acid, ferulic acid, thiotaurine, hypotaurine, sulfites, erythorbic acid and salts thereof, chlorogenic acid, epicatechin, epigallocatechin, epigallocatechin gallate, apigenin, kaempferol, myricetin, and quercetin. The antioxidants may be used singly, or two or more may be used in combination.

### • Acidity regulators

Examples of the acidity regulators include lactic acid, citric acid, glycolic acid, succinic acid, tartaric acid, dl-malic acid, potassium carbonate, sodium hydrogencarbonate, and ammonium hydrogencarbonate.

### • Chelating agents

Examples of the chelating agents include alanine, sodium EDTA, sodium polyphosphate, sodium metaphosphate, and phosphoric acid.

### • Refreshing agents

Examples of the refreshing agents include L-menthol, camphor, and menthyl lactate.

### • Anti-inflammatory agents

Examples of the anti-inflammatory agents include allantoin, glycyrrhizinic acid and salts thereof, glycyrrhetinic acid, stearyl glycyrrhetinate, tranexamic acid, and azulene.

### • Skin modifying ingredients

Examples of the skin modifying ingredients include brightening or whitening agents, such as placenta extract, arbutin, glutathione, and Saxifraga Sarmentosa Extract; cell activators, such as royal jelly, photosensitizers, cholesterol derivatives, and bovine blood extracts; anti-skin-roughening agents; blood flow enhancing agents, such as vanillylamide nonylate, benzyl nicotinate, β-butoxyethyl nicotinate, capsaicin, zingerone, cantharides tincture, ichthammol, caffeine, tannic acid, α-borneol, tocopherol nicotinate, inositol hexanicotinate, cyclandelate, cinnarizine, tolazoline, acetylcholine, verapamil, cepharanthine, and γ-oryzanol; skin astringents, such as zinc oxide and tannic acid; and antiseborrheic agents, such as sulfur and thianthol.

### • Vitamins

Examples of the vitamins include vitamin A family, such as vitamin A oil, retinol, retinol acetate, and retinol palmitate; vitamin B family, specifically, vitamin B2 family, such as riboflavin, riboflavin butyrate, and flavin adenine nucleotide, vitamin B6 family, such as pyridoxine hydrochloride, pyridoxine dioctanoate, and pyridoxine tripalmitate, vitamin B12 and derivatives thereof, and vitamin B15 and derivatives thereof; vitamin C family, such as L-ascorbic acid, L-ascorbic acid dipalmitic acid ester, L-ascorbic acid-2-sodium sulfate, and dipotassium L-ascorbic acid phosphoric acid diester; vitamin D family, such as ergocalciferol and cholecalciferol; vitamin E family, such as α-tocopherol, β-tocopherol, γ-tocopherol, dl-α-tocopherol acetate, dl-α-tocopherol nicotinate, and dl-α-tocopherol succinate; nicotinic acids, such as nicotinic acid, benzyl nicotinate, and nicotinic amide; vitamin H; vitamin P; pantothenic acids, such as calcium pantothenate, D-pantothenyl alcohol, pantothenyl ethyl ether, and acetylpantothenyl ethyl ether; and biotin.

### • Amino acids

Examples of the amino acids include glycine, valine, leucine, isoleucine, serine, threonine, phenylalanine, arginine, lysine, aspartic acid, glutamic acid, cystine, cysteine, methionine, and tryptophan.

### • Nucleic acids

Examples of the nucleic acids include deoxyribonucleic acid.

### • Hormones

Examples of the hormones include estradiol and ethenyl estradiol.

### • Clathrate compounds

Examples of the clathrate compounds include cyclodextrin.

### EXAMPLES

The present invention will be described in detail below by presenting Production Examples, Comparative Production Example, and Examples and Comparative Examples of cosmetics. However, it should be construed that the present invention is not limited to the following Examples. Unless otherwise specified, the compositional "%" described below is "mass%" and means the mass percentage of the ingredient relative to the mass of the whole in each Example taken as 100 mass%. The amounts are the amounts of the products described. In the following Production Examples 1 to 11, the kinematic viscosity is a value measured at 25°C using a Cannon-Fenske viscometer as described in JIS Z8803: 2011.

### [Production Example 1]

An autoclave was charged with 100 parts by mass of an amino-modified silicone represented by formula (5) below, 14 parts by mass of propylene oxide, and 6 parts by mass of hexylene glycol. The mixture was heated at 130°C for 6 hours. Unreacted materials were stripped from the resultant reaction mixture under reduced pressure at 130°C. Thus, a pale yellow, transparent liquid with a kinematic viscosity of 1,000 mm²/s was obtained. ¹H-NMR (CDCl₃, 400 MHz, Bruker) measurement confirmed that at least 99 mol% of the -NH₂ groups had been converted to -N(CH₂-CH(OH)-CH₃) or -N(CH(CH₃)-CH₂OH).

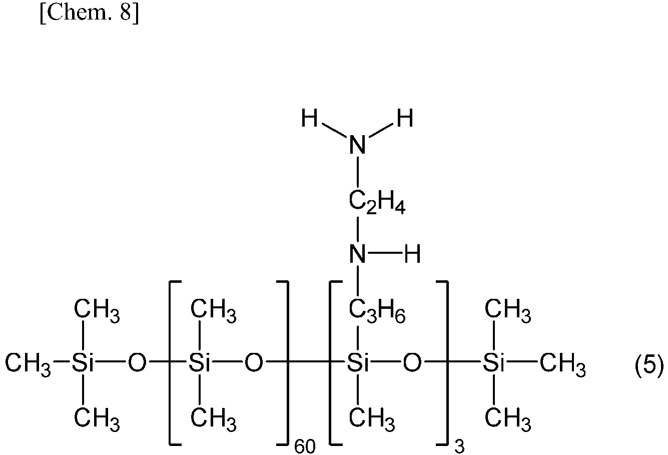

### [Production Example 2]

An autoclave was charged with 100 parts by mass of an amino-modified silicone represented by formula (6) below, 11 parts by mass of propylene oxide, and 6 parts by mass of hexylene glycol. The mixture was heated at 130°C for 6 hours. Unreacted materials were stripped from the resultant reaction mixture under reduced pressure at 130°C. Thus, a pale yellow, transparent liquid with a kinematic viscosity of 510 mm²/s was obtained. ¹H-NMR (CDCl₃, 400 MHz, Bruker) measurement confirmed that at least 99 mol% of the -NH₂ groups had been converted to -N(CH₂-CH(OH)-CH₃) or -N(CH(CH₃)-CH₂OH).

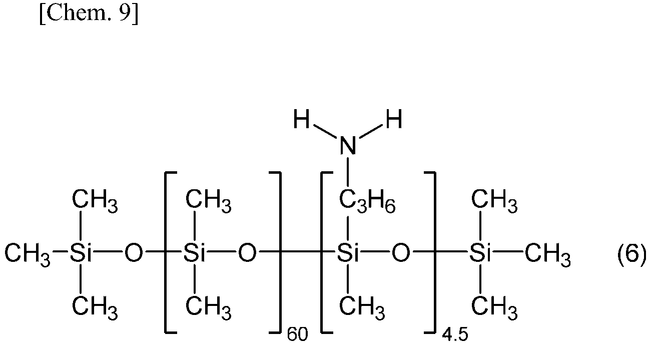

### [Production Example 3]

An autoclave was charged with 100 parts by mass of an amino-modified silicone represented by formula (7) below, 20 parts by mass of propylene oxide, and 6 parts by mass of hexylene glycol. The mixture was heated at 130°C for 6 hours. Unreacted materials were stripped from the resultant reaction mixture under reduced pressure at 130°C. Thus, a pale yellow, transparent liquid with a kinematic viscosity of 900 mm²/s was obtained. ¹H-NMR (CDCl₃, 400 MHz, Bruker) measurement confirmed that at least 99 mol% of the -NH₂ groups had been converted to -N(CH₂-CH(OH)-CH₃) or -N(CH(CH₃)-CH₂OH).

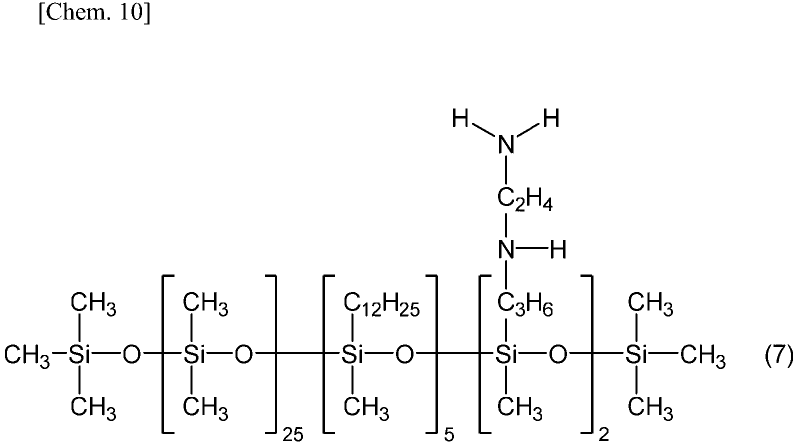

### [Production Example 4]

An autoclave was charged with 100 parts by mass of an amino-modified silicone represented by formula (8) below, 16 parts by mass of propylene oxide, and 6 parts by mass of hexylene glycol. The mixture was heated at 130°C for 6 hours. Unreacted materials were stripped from the resultant reaction mixture under reduced pressure at 130°C. Thus, a pale yellow, transparent liquid with a kinematic viscosity of 260 mm²/s was obtained. ¹H-NMR (CDCl₃, 400 MHz, Bruker) measurement confirmed that at least 99 mol% of the -NH₂ groups had been converted to -N(CH₂-CH(OH)-CH₃) or -N(CH(CH₃)-CH₂-OH).

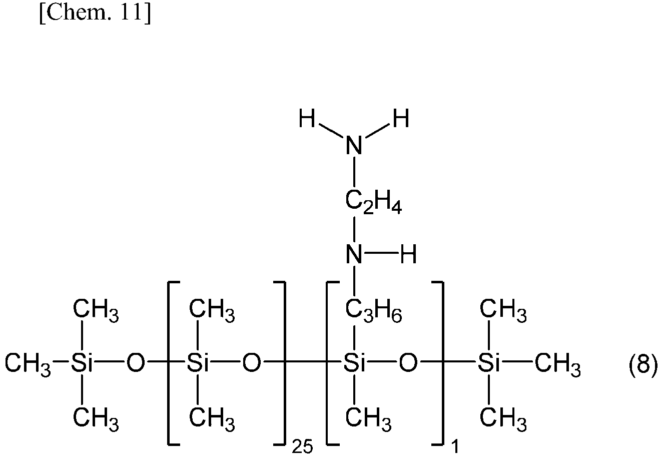

### [Production Example 5]

An autoclave was charged with 100 parts by mass of an amino-modified silicone represented by formula (9) below, 8 parts by mass of propylene oxide, and 6 parts by mass of hexylene glycol. The mixture was heated at 130°C for 6 hours. Unreacted materials were stripped from the resultant reaction mixture under reduced pressure at 130°C. Thus, a pale yellow, transparent liquid with a kinematic viscosity of 3,200 mm²/s was obtained. ¹H-NMR (CDCl₃, 400 MHz, Bruker) measurement confirmed that 60 mol% of the -NH₂ groups had been converted to -N(CH₂-CH(OH)-CH₃) or -N(CH(CH₃)-CH₂-OH).

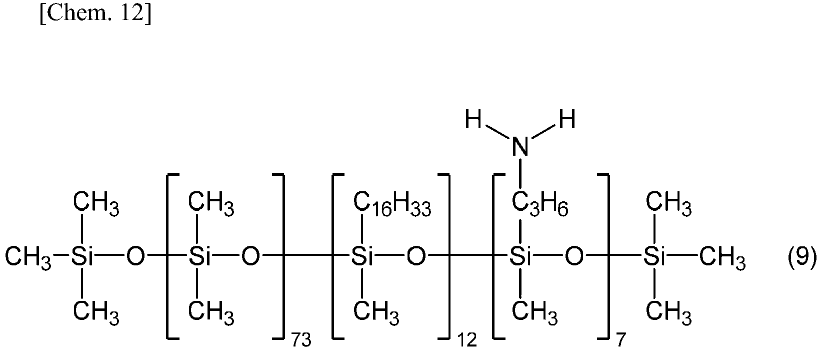

### [Production Example 6]

An autoclave was charged with 100 parts by mass of an amino-modified silicone represented by formula (10) below, 10 parts by mass of propylene oxide, and 6 parts by mass of hexylene glycol. The mixture was heated at 130°C for 6 hours. Unreacted materials were stripped from the resultant reaction mixture under reduced pressure at 130°C. Thus, a pale yellow, transparent liquid with a kinematic viscosity of 1,300 mm²/s was obtained. ¹H-NMR (CDCl₃, 400 MHz, Bruker) measurement confirmed that at least 99 mol% of the -NH₂ groups had been converted to -N(CH₂-CH(OH)-CH₃) or -N(CH(CH₃)-CH₂-OH).

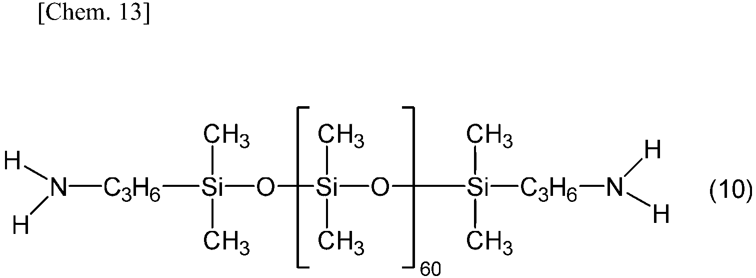

### [Production Example 7]

An autoclave was charged with 100 parts by mass of an amino-modified silicone represented by formula (11) below, 11 parts by mass of propylene oxide, and 4 parts by mass of hexylene glycol. The mixture was heated at 130°C for 6 hours. Unreacted materials were stripped from the resultant reaction mixture under reduced pressure at 130°C. Thus, a pale yellow, transparent liquid with a kinematic viscosity of 1,800 mm²/s was obtained. ¹H-NMR (CDCl₃, 400 MHz, Bruker) measurement confirmed that at least 99 mol% of the -NH₂ groups had been converted to -N(CH₂-CH(OH)-CH₃) or -N(CH(CH₃)-CH₂-OH).

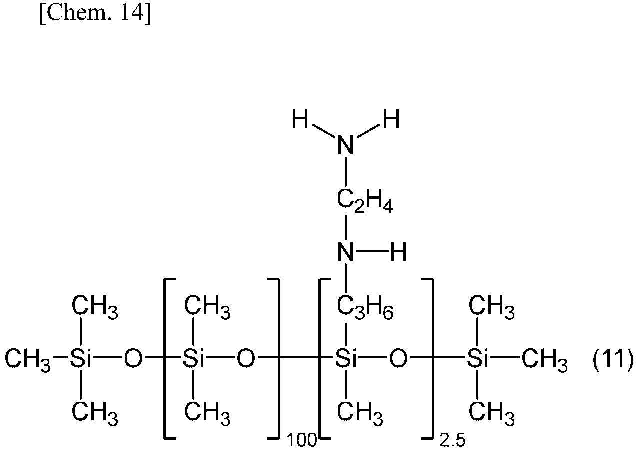

### [Production Example 8]

An autoclave was charged with 100 parts by mass of an amino-modified silicone represented by formula (12) below, 60 parts by mass of propylene oxide, and 6 parts by mass of hexylene glycol. The mixture was heated at 130°C for 6 hours. Unreacted materials were stripped from the resultant reaction mixture under reduced pressure at 130°C. Thus, a pale yellow, transparent liquid with a kinematic viscosity of 1,100 mm²/s was obtained. ¹H-NMR (CDCl₃, 400 MHz, Bruker) measurement confirmed that at least 99 mol% of the -NH₂ groups had been converted to -N(CH₂-CH(OH)-CH₃) or -N(CH(CH₃)-CH₂-OH).

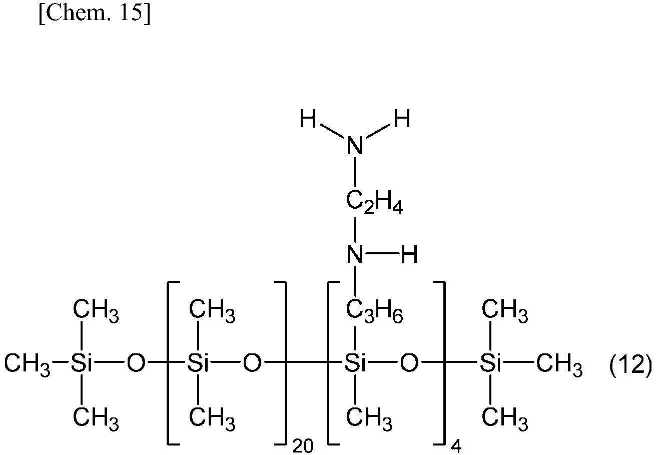

### [Production Example 9]

An autoclave was charged with 100 parts by mass of an amino-modified silicone represented by formula (13) below, 14 parts by mass of propylene oxide, and 6 parts by mass of hexylene glycol. The mixture was heated at 130°C for 6 hours. Unreacted materials were stripped from the resultant reaction mixture under reduced pressure at 130°C. Thus, a pale yellow, transparent liquid with a kinematic viscosity of 760 mm²/s was obtained. ¹H-NMR (CDCl₃, 400 MHz, Bruker) measurement confirmed that at least 99 mol% of the -NH₂ groups had been converted to -N(CH₂-CH(OH)-CH₃) or -N(CH(CH₃)-CH₂-OH).

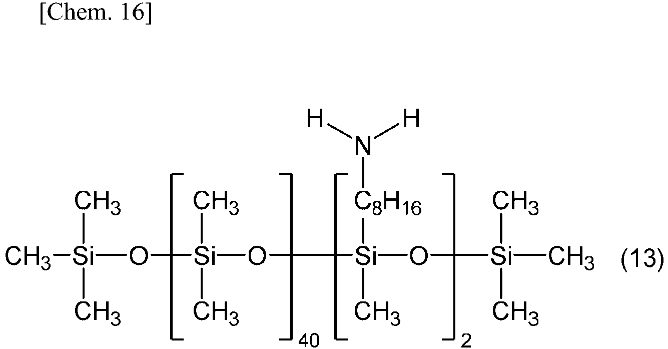

### [Production Example 10]

An autoclave was charged with 100 parts by mass of an amino-modified silicone represented by formula (14) below, 26 parts by mass of propylene oxide, and 6 parts by mass of hexylene glycol. The mixture was heated at 130°C for 6 hours. Unreacted materials were stripped from the resultant reaction mixture under reduced pressure at 130°C. Thus, a pale yellow, transparent liquid with a kinematic viscosity of 480 mm²/s was obtained. ¹H-NMR (CDCl₃, 400 MHz, Bruker) measurement confirmed that at least 99 mol% of the -NH₂ groups had been converted to -N(CH₂-CH(OH)-CH₃) or -N(CH(CH₃)-CH₂-OH).

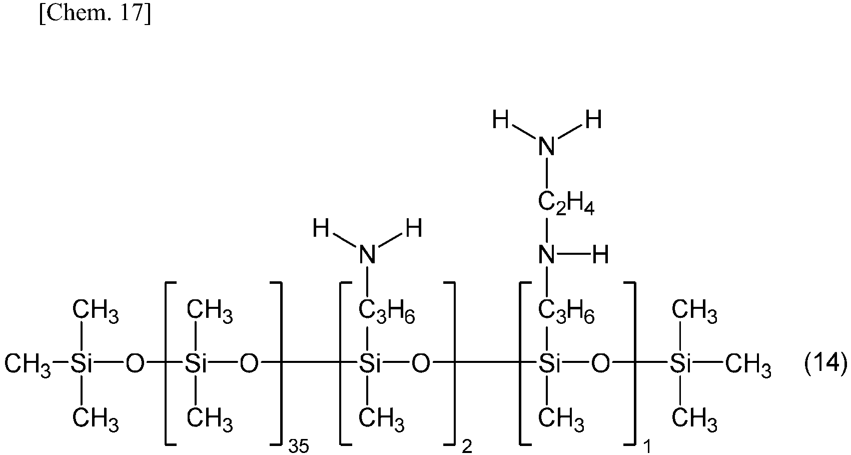

### [Production Example 11]

An autoclave was charged with 100 parts by mass of an amino-modified silicone represented by formula (15) below, 20 parts by mass of propylene oxide, and 6 parts by mass of hexylene glycol. The mixture was heated at 130°C for 6 hours. Unreacted materials were stripped from the resultant reaction mixture under reduced pressure at 130°C. Thus, a pale yellow, transparent liquid with a kinematic viscosity of 1,500 mm²/s was obtained. ¹H-NMR (CDCl₃, 400 MHz, Bruker) measurement confirmed that at least 99 mol% of the -NH₂ groups had been converted to -N(CH₂-CH(OH)-CH₃) or -N(CH(CH₃)-CH₂-OH).

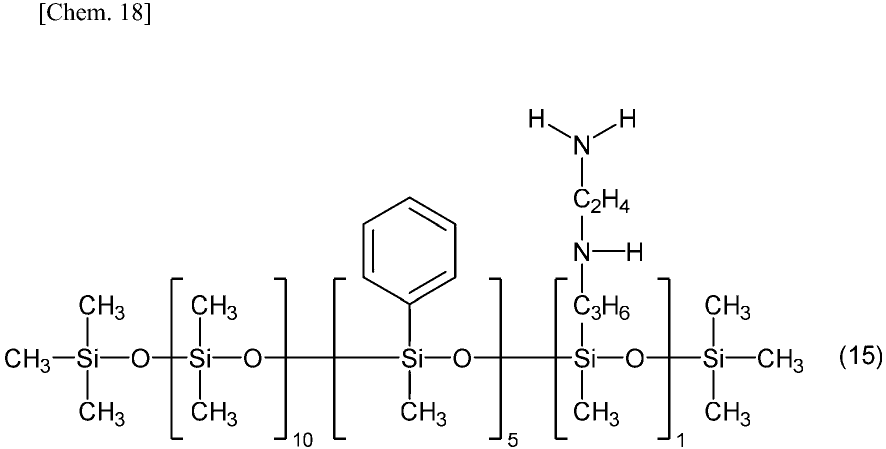

### [Comparative Production Example 1]

An autoclave was charged with 100 parts by mass of an amino-modified silicone represented by formula (16) below, 7 parts by mass of propylene oxide, and 6 parts by mass of hexylene glycol. The mixture was heated at 130°C for 6 hours. Unreacted materials were stripped from the resultant reaction mixture under reduced pressure at 130°C. Thus, a pale yellow, transparent liquid with a kinematic viscosity of 1,400 mm²/s was obtained. ¹H-NMR (CDCl₃, 400 MHz, Bruker) measurement confirmed that at least 99 mol% of the -NH₂ groups had been converted to -N(CH₂-CH(OH)-CH₃) or -N(CH(CH₃)-CH₂-OH).

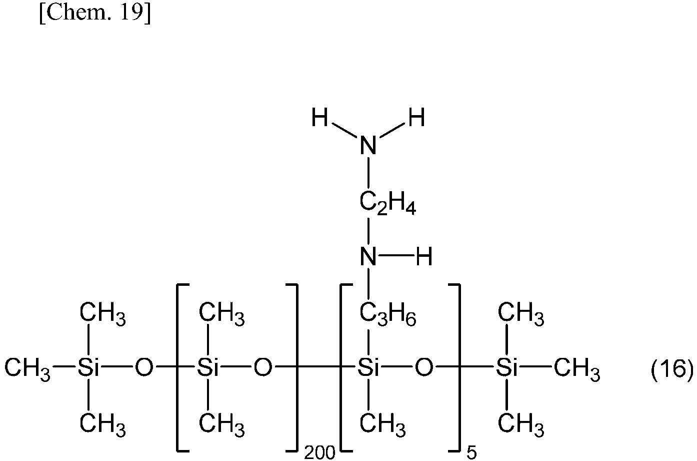

The advantageous effects of the present invention will be demonstrated below by way of Examples and Comparative Examples related to cosmetics.

### [Examples 1 to 4, and Comparative Examples 1 to 5]

Water-in-oil (W/O) cosmetics were prepared according to the formulations described in Table 1. The water-in-oil (W/O) cosmetics obtained were evaluated as follows.

### (Production method)

- A:: Ingredients (1) were mixed.
- B:: Ingredients (2) were mixed.
- C:: The mixture obtained in B was added to the mixture obtained in A and emulsified to give a water-in-oil (W/O) cosmetic. Except the emulsion obtained in Comparative Example 5, the samples having been added to a height of 1 cm from the mouth of a 30 mL vial exhibited fluidity, namely, started to flow at 25°C within one minute after the vial was placed on its side.

### [Emulsion particle size]

The cosmetic obtained was observed under an optical microscope, and the sizes of 100 emulsion particles were measured and averaged. The rating was "A" when the average particle size was less than 2.0 µm, "B" when the average particle size was 2.0 or more and less than 4.0 µm, "C" when the average particle size was 4.0 or more and less than 6.0 µm, and "D" when the average particle size was 6.0 µm or more. Those cosmetics that had "C" or higher rating (A, B, and C) were accepted.

### [Feeling on use]

The feeling on use (low stickiness) of the cosmetics was evaluated by a panel of ten experts based on the scoring criteria below. The scores of the ten experts were averaged and the results were evaluated according to the evaluation criteria below. The results are described in the table.

### [Scoring criteria]

| | |
|---|---|
| 5 Points: | Very good |
| 4 Points: | Good |
| 3 Points: | Fair |
| 2 Points: | Rather poor |
| 1 Point: | Poor |

The average score was evaluated based on the following criteria.

### [Evaluation criteria]

- A:: The average score is 4.5 points or more.
- B:: The average score is 3.5 points or more and less than 4.5 points.
- C:: The average score is 2.5 points or more and less than 3.5 points.
- D:: The average score is 1.5 points or more and less than 2.5 points.
- E:: The average score is less than 1.5 points.

Those cosmetics that had "C" or higher rating (A, B, and C) were accepted.

### [Viscosity stability]

The cosmetic was added into a 30 mL vial and stored in a thermostatic chamber at 50°C for one week. The viscosity was then measured.

The viscosity after storage (the post-storage viscosity) was compared to the viscosity immediately after preparation (the initial viscosity) and the viscosity change (%) was calculated from (post-storage viscosity/initial viscosity - 1) × 100. The viscosity stability was rated as "A" when the viscosity change was 0% or more and less than ±10%, "B" when the viscosity change was ±10% or more and less than ±15%, "C" when the viscosity change was ±15% or more and less than ±20%, "D" when the viscosity change was ±20% or more and less than ±30%, and "E" when the cosmetic had separated or the viscosity change was ±30% or more. Those cosmetics that had "C" or higher rating were accepted. The viscosity for this evaluation was measured with a Brookfield viscometer (TVB-10, manufactured by Toki Sangyo Co., Ltd.) with spindle No. 4, at 6 rpm for a measurement time of 60 seconds.

**[Table 1]**

| Composition (%) | | Example | | | | Comparative Example | | | | |
|---|---|---|---|---|---|---|---|---|---|---|
| | | 1 | 2 | 3 | 4 | 1 | 2 | 3 | 4 | 5 |
| (1) | Production Example 1 | 2 | 2 | 2 | - | 2 | - | - | - | - |
| | Production Example 2 | - | - | - | 2 | - | 2 | - | - | - |
| | Comparative Production Example 1 | - | - | - | - | - | - | - | - | 2 |
| | KF-6017 (Note 1) | - | - | - | - | - | - | 2 | - | - |
| | KF-6104 (Note 2) | - | - | - | - | - | - | - | 2 | - |
| | KF-96A-6cs (Note 3) | 23 | 23 | 23 | 23 | 23 | 23 | 23 | 23 | 23 |
| (2) | Sodium chloride | 0.6 | 1 | - | 1 | 0.5 | 0.5 | 0.5 | 1 | - |
| | Magnesium sulfate | - | - | 1 | - | - | - | - | - | 1 |
| | Butylene Glycol | 5 | 5 | 5 | 5 | 5 | 5 | 5 | 5 | 5 |
| | Water | 69.4 | 69.0 | 69.0 | 69.0 | 69.5 | 69.5 | 69.5 | 69.0 | 69.0 |
| Total | | 100.0 | 100.0 | 100.0 | 100.0 | 100.0 | 100.0 | 100.0 | 100.0 | 100.0 |
| Emulsion particle size | | B | B | B | B | B | B | B | D | B |
| Feeling on use | | A | A | A | A | A | A | A | B | E |
| Viscosity stability | | C | B | A | A | D | E | E | D | A |

| | | | | | | | | | | |
|---|---|---|---|---|---|---|---|---|---|---|
| Production Example 1: Aminoalcohol-modified silicone obtained in Production Example 1 Production Example 2: Aminoalcohol-modified silicone obtained in Production Example 2 (Note 1) PEG-10 dimethicone, Shin-Etsu Chemical Co., Ltd. (Note 2) Polyglyceryl-3 polydimethylsiloxyethyl dimethicone, Shin-Etsu Chemical Co., Ltd. (Note 3) Dimethicone (6 cs), Shin-Etsu Chemical Co., Ltd. | | | | | | | | | | |

As is clear from Table 1, the cosmetics of Examples 1 to 4 had a small emulsion particle size and achieved good emulsion viscosity stability even without containing a thickening agent.

### [Examples 5 and 6, and Comparative Examples 6 to 9]

Water-in-oil (W/O) cosmetics were prepared according to the formulations described in Table 2. The water-in-oil (W/O) cosmetics obtained were evaluated as follows.

### (Production method)

- A:: Ingredients (1) were mixed.
- B:: Ingredients (2) were mixed.
- C:: The mixture obtained in B was added to the mixture obtained in A and emulsified to give a water-in-oil (W/O) cosmetic.

**[Table 2]**

| Composition (%) | | Example | | Comparative Example | | | |
|---|---|---|---|---|---|---|---|
| | | 5 | 6 | 6 | 7 | 8 | 9 |
| (1) | Production Example 2 | 2 | 2 | 2 | 2 | - | - |
| | Comparative Production Example 1 | - | - | - | - | - | 2 |
| | KF-6104 (Note 1) | - | - | - | - | 2 | - |
| | KF-96A-6cs (Note 2) | 23 | - | 23 | - | - | - |
| | Isotridecyl isononanoate | - | 23 | - | 23 | 23 | 23 |
| (2) | Sodium chloride | 1 | 1 | 0.5 | 0.5 | 1 | 1 |
| | Ethanol | 5 | 5 | 5 | 5 | 5 | 5 |
| | Butylene Glycol | 5 | 5 | 5 | 5 | 5 | 5 |
| | Water | 64 | 64 | 64.5 | 64.5 | 64 | 64 |
| Total | | 100.0 | 100.0 | 100.0 | 100.0 | 100.0 | 100.0 |
| Emulsion particle size | | B | B | B | B | D | B |
| Feeling on use | | A | B | A | A | B | E |
| Viscosity stability | | C | A | E | E | D | A |

| | | | | | | | |
|---|---|---|---|---|---|---|---|
| Production Example 2: Aminoalcohol-modified silicone obtained in Production Example 2 Comparative Production Example 1: Aminoalcohol-modified silicone obtained in Comparative Production Example 1 (Note 1) Polyglyceryl-3 polydimethylsiloxyethyl dimethicone, Shin-Etsu Chemical Co., Ltd. (Note 2) Dimethicone (6 cs), Shin-Etsu Chemical Co., Ltd. | | | | | | | |

As is clear from Table 2, the cosmetics of Examples 5 and 6 had a small emulsion particle size and had good emulsion viscosity stability in spite of the fact that the emulsions contained ethanol. Further Examples are described below. The following Examples also achieved similar emulsion particle sizes and emulsion viscosity stability to Examples 1 to 6.

### [Example 7] Water-in-oil sunscreen cream

| | Composition | % |
|---|---|---|
| 1. | Cyclopentasiloxane | 30 |
| 2. | Squalane | 2.5 |
| 3. | Aminoalcohol-modified silicone obtained in Production Example 1 | 1.5 |
| 4. | KF-6038 (Note 1) | 3 |
| 5. | Ethylhexyl methoxycinnamate | 7.5 |
| 6. | t-Butyl methoxydibenzoylmethane | 3 |
| 7. | Polysilicone-15 | 1 |
| 8. | Disteardimonium hectorite | 1 |
| 9. | Tocopherol acetate | 0.1 |
| 10. | Ethanol | 1 |
| 11. | Butylene Glycol | 3.5 |
| 12. | Sodium citrate | 0.5 |
| 13. | Magnesium sulfate | 0.6 |
| 14. | Preservative | 0.3 |
| 15. | Water | Balance |
| Total | | 100.0 |

| | | |
|---|---|---|
| (Note 1) Lauryl PEG-9 polydimethylsiloxyethyl dimethicone, Shin-Etsu Chemical Co., Ltd. | | |

### (Production method)

- A:: Ingredients 1 to 9 were mixed uniformly.
- B:: Ingredients 10 to 14 were dissolved uniformly into ingredient 15.
- C:: While performing stirring, the mixture obtained in B was gradually added to the mixture obtained in A and emulsified to give a water-in-oil sunscreen cream.

The water-in-oil sunscreen cream obtained as described above had a very good feeling on use and was also excellent in emulsion viscosity stability without any changes due to temperature or aging.

### [Example 8] Water-in-oil lip cream

| | Composition | % |
|---|---|---|
| 1. | Dextrin (palmitate/ethylhexanoate) (Note 1) | 9 |
| 2. | Cyclopentasiloxane | 53 |
| 3. | KP-545 (Note 2) | 5 |
| 4. | KSG-43 (Note 3) | 8 |
| 5. | Aminoalcohol-modified silicone obtained in Production Example 1 | 0.5 |
| 6. | KF-6038 (Note 4) | 1.5 |
| 7. | Butylene Glycol | 5 |
| 8. | Water | 10.2 |
| 9. | Sodium chloride | 0.6 |
| 10. | Colorant | 1.2 |
| 11. | Titanium dioxide-coated mica | 6 |
| Total | | 100.0 |

| | | |
|---|---|---|
| (Note 1) Rheopearl TT, Chiba Flour Milling Co., Ltd. (Note 2) Solution of 70% cyclopentasiloxane + 30% (acrylates/dimethicone) copolymer, Shin-Etsu Chemical Co., Ltd. (Note 3) Mixture of 65-75% triethylhexanoin + 25-35% (vinyl dimethicone/lauryl dimethicone) crosspolymer, Shin-Etsu Chemical Co., Ltd. (Note 4) Lauryl PEG-9 polydimethylsiloxyethyl dimethicone, Shin-Etsu Chemical Co., Ltd. | | |

### (Production method)

- A:: Ingredient 10 was mixed with part of ingredient 2 and dispersed with a roller mill. The dispersion obtained was heated and mixed together with ingredient 1, the remainder of ingredient 2, and ingredients 3 to 6.
- B:: Ingredients 7, 8, and 9 were heated and added to the mixture obtained in A. The resultant mixture was emulsified and then cooled.
- C:: Ingredient 11 was added to the emulsion obtained in B to give a water-in-oil lip cream.

The water-in-oil lip cream obtained spread lightly, was not sticky or oily, and formed a long-lasting film on the lips.

### [Example 9] Water-in-oil sunscreen milky lotion

| | Composition | % |
|---|---|---|
| 1. | KSG-210 (Note 1) | 3 |
| 2. | KSG-15 (Note 2) | 2 |
| 3. | Aminoalcohol-modified silicone obtained in Production Example 2 | 1 |
| 4. | Dimethicone 6 CS | 5 |
| 5. | Cyclopentasiloxane | 36 |
| 6. | Isotridecyl isononanoate | 4 |
| 7. | Hydrophobized microparticulate titanium dioxide | 15 |
| 8. | Hydrophobized microparticulate zinc oxide | 10 |
| 9. | Silica | 0.2 |
| 10. | Dipropylene Glycol | 5 |
| 11. | Sodium citrate | 0.2 |
| 12. | Sodium chloride | 1 |
| 13. | Preservative | q.s. |
| 14. | Fragrance | q.s. |
| 15. | Water | Balance |
| | Total | 100.0 |

| | | |
|---|---|---|
| (Note 1) Mixture of 70-80% dimethicone + 20-30% (dimethicone/(PEG-10/15)) crosspolymer, Shin-Etsu Chemical Co., Ltd. (Note 2) Mixture of 90-96% cyclopentasiloxane + 4-10% (dimethicone/vinyl dimethicone) crosspolymer, Shin-Etsu Chemical Co., Ltd. | | |

### (Production method)

- A:: Ingredients 7 and 8 were mixed with part of ingredient 5 and part of ingredient 15, and the mixture was uniformly dispersed with a bead mill.
- B:: Ingredients 1 to 4, the remainder of ingredient 5, and ingredients 6 and 9 were mixed uniformly.
- C:: Ingredients 10 to 13 and the remainder of ingredient 15 were mixed.
- D:: While performing stirring, the mixture obtained in C was added to the mixture obtained in B and emulsified. The mixture obtained in A and ingredient 14 were further added to give a water-in-oil sunscreen milky lotion.

The water-in-oil sunscreen milky lotion obtained as described above had a very good feeling on use and was also excellent in emulsion viscosity stability without any changes due to temperature or aging.

### [Example 10] Water-in-oil sunscreen cream

| | Composition | % |
|---|---|---|
| 1. | KSG-340 (Note 1) | 3 |
| 2. | KSG-43 (Note 2) | 3 |
| 3. | Aminoalcohol-modified silicone obtained in Production Example 3 | 1.5 |
| 4. | KF-56A (Note 3) | 11 |
| 5. | Ethylhexyl methoxycinnamate | 7 |
| 6. | KSP-100 (Note 4) | 2 |
| 7. | Xanthan gum | 0.3 |
| 8. | Dipropylene Glycol | 5 |
| 9. | Glycerin | 3 |
| 10. | Methylparaben | 0.1 |
| 11. | Sodium citrate | 0.2 |
| 12. | Sodium chloride | 1.5 |
| 13. | Water | Balance |
| | Total | 100.0 |

| | | |
|---|---|---|
| (Note 1) Mixture of 65-75% squalane + 25-35% (PEG-10/lauryl dimethicone) crosspolymer and (PEG-15/lauryl dimethicone) crosspolymer, Shin-Etsu Chemical Co., Ltd. (Note 2) Mixture of 65-75% triethylhexanoin + 25-35% (vinyl dimethicone/lauryl dimethicone) crosspolymer, Shin-Etsu Chemical Co., Ltd. (Note 3) Diphenylsiloxyphenyl trimethicone, Shin-Etsu Chemical Co., Ltd. (Note 4) (Vinyl dimethicone/methicone silsesquioxane) crosspolymer, Shin-Etsu Chemical Co., Ltd. | | |

### (Production method)

- A:: Ingredients 1 to 6 were mixed uniformly.
- B:: Ingredients 7 to 13 were mixed uniformly.
- C:: The mixture obtained in B was added to the mixture obtained in A and emulsified to give a water-in-oil sunscreen cream.

The water-in-oil sunscreen cream obtained as described above had a very good feeling on use and was also excellent in emulsion viscosity stability without any changes due to temperature or aging.

### [Example 11] Water-in-oil foundation

| | Composition | % |
|---|---|---|
| 1. | KSG-210 (Note 1) | 3.5 |
| 2. | KSG-15 (Note 2) | 5 |
| 3. | Aminoalcohol-modified silicone obtained in Production Example 4 | 2 |
| 4. | Disteardimonium hectorite | 1.2 |
| 5. | Triethylhexanoin | 5 |
| 6. | Dimethicone 6 CS | 6.5 |
| 7. | Cyclopentasiloxane | 22.6 |
| 8. | KP-578 (Note 3) | 0.5 |
| 9. | KTP-09W (Note 4) | q.s. |
| 10. | KTP-09R (Note 4) | q.s. |
| 11. | KTP-09Y (Note 4) | q.s. |
| 12. | KTP-09B (Note 4) | q.s. |
| 13. | Butylene Glycol | 5 |
| 14. | Sodium citrate | 0.2 |
| 15. | Sodium chloride | 2 |
| 16. | Water | 36.5 |
| | Total | 100.0 |

| | | |
|---|---|---|
| (Note 1) Mixture of 70-80% dimethicone + 20-30% (dimethicone/(PEG-10/15)) crosspolymer, Shin-Etsu Chemical Co., Ltd. (Note 2) Mixture of 90-96% cyclopentasiloxane + 4-10% (dimethicone/vinyl dimethicone) crosspolymer, Shin-Etsu Chemical Co., Ltd. (Note 3) (Acrylates/ethylhexyl acrylate/dimethicone methacrylate) copolymer, Shin-Etsu Chemical Co., Ltd. (Note 4) KF-9909-treated coloring inorganic pigments, W: white, R: red, Y: yellow, B: black, Shin-Etsu Chemical Co., Ltd. | | |

### (Production method)

- A:: Ingredients 8 to 12 and part of ingredient 7 were uniformly dispersed with a roll mill.
- B:: Ingredients 1 to 6 and the remainder of ingredient 7 were mixed uniformly.
- C:: Ingredients 13 to 16 were mixed uniformly.
- D:: The mixture obtained in C was added to the mixture obtained in B and emulsified. The mixture obtained in A was further added and mixed to give a water-in-oil foundation.

The water-in-oil foundation obtained as described above had a very good feeling on use and was also excellent in stability without any changes due to temperature or aging.

### [Example 12] Water-in-oil stick foundation

| | Composition | % |
|---|---|---|
| 1. | KSG-210 (Note 1) | 4 |
| 2. | Aminoalcohol-modified silicone obtained in Production Example 5 | 1.5 |
| 3. | Inulin stearate (Note 2) | 2 |
| 4. | Ceresin | 6 |
| 5. | KF-56A (Note 3) | 11.5 |
| 6. | Ethylhexyl methoxycinnamate | 5 |
| 7. | KMP-590 (Note 4) | 1.5 |
| 8. | Isotridecyl isononanoate | 4 |
| 9. | KF-6048 (Note 5) | 1 |
| 10. | KTP-09W (Note 6) | 6.5 |
| 11. | KTP-09R (Note 6) | q.s. |
| 12. | KTP-09Y (Note 6) | q.s. |
| 13. | KTP-09B (Note 6) | q.s. |
| 14. | Dipropylene Glycol | 6 |
| 15. | Methylparaben | 0.1 |
| 16. | Sodium citrate | 0.2 |
| 17. | Magnesium chloride | 1 |
| 18. | Water | Balance |
| | Total | 100.0 |

| | | |
|---|---|---|
| (Note 1) Mixture of 70-80% dimethicone + 20-30% (dimethicone/(PEG-10/15)) crosspolymer, Shin-Etsu Chemical Co., Ltd. (Note 2) Rheopearl ISK2, Chiba Flour Milling Co., Ltd. (Note 3) Diphenylsiloxyphenyl trimethicone, Shin-Etsu Chemical Co., Ltd. (Note 4) Polymethylsilsesquioxane, Shin-Etsu Chemical Co., Ltd. (Note 5) Cetyl PEG/PPG-10/1 dimethicone, Shin-Etsu Chemical Co., Ltd. (Note 6) KF-9909-treated coloring inorganic pigments, W: white, R: red, Y: yellow, B: black, Shin-Etsu Chemical Co., Ltd. | | |

### (Production method)

- A:: Ingredients 8 to 13 were dispersed with a roll mill.
- B:: The dispersion obtained in A and ingredients 1 to 7 were heated to 95°C and mixed uniformly.
- C:: Ingredients 14 to 18 were mixed uniformly and heated to 85°C.
- D:: The mixture obtained in C was added to the mixture obtained in B and emulsified. The emulsion obtained was poured into a stick container and slowly cooled to give a water-in-oil stick foundation.

The water-in-oil stick foundation obtained as described above had a good feeling on use, spread well, and exhibited good cosmetic durability and emulsion viscosity stability.

### [Example 13] Polyhydric alcohol-in-oil emulsion cream

| | Composition | % |
|---|---|---|
| 1. | KSG-240 (Note 1) | 5 |
| 2. | KSG-15 (Note 2) | 20 |
| 3. | KSG-16 (Note 3) | 35 |
| 4. | Aminoalcohol-modified silicone obtained in Production Example 6 | 1 |
| 5. | Cyclopentasiloxane | 5 4 |
| 6. 10. | KF-7312J (Note 4) | |
| 7. | KSP-300 (Note 5) | 5 |
| 8. | Preservative | q.s. |
| 9. | Fragrance | q.s. |
| | Sodium chloride | 0.6 |
| 11. | Glycerin | 5 |
| 12. | Butylene Glycol | 10 |
| | Total | 100.0 |

| | | |
|---|---|---|
| (Note 1) Mixture of 75-85% cyclopentasiloxane + 15-25% (dimethicone/(PEG-10/15)) crosspolymer, Shin-Etsu Chemical Co., Ltd. (Note 2) Mixture of 90-96% cyclopentasiloxane + 4-10% (dimethicone/vinyl dimethicone) crosspolymer, Shin-Etsu Chemical Co., Ltd. (Note 3) Mixture of 70-80% dimethicone + 20-30% (dimethicone/vinyl dimethicone) crosspolymer, Shin-Etsu Chemical Co., Ltd. (Note 4) Cyclopentasiloxane solution of 50% trimethylsiloxysilicic acid, Shin-Etsu Chemical Co., Ltd. (Note 5) (Diphenyl dimethicone/vinyl diphenyl dimethicone/silsesquioxane) crosspolymer, Shin-Etsu Chemical Co., Ltd. | | |

### (Production method)

- A:: Ingredients 1 to 9 were admixed uniformly.
- B:: Ingredients 8 and 10 to 12 were mixed.
- C:: The mixture obtained in B was added to the mixture obtained in A and emulsified uniformly to give a polyhydric alcohol-in-oil emulsion cream.

The polyhydric alcohol-in-oil emulsion cream obtained as described above spread lightly, was not sticky or oily, and had a moist feel.

### [Example 14] Water-in-oil cream

| | Composition | % |
|---|---|---|
| 1. | KSG-210 (Note 1) | 4 |
| 2. | Aminoalcohol-modified silicone obtained in Production Example 7 | 0.5 |
| 3. | Cyclopentasiloxane | 8.8 |
| 4. | KSP-101 (Note 2) | 2 |
| 5. | Sodium chloride | 0.7 |
| 6. | Butylene Glycol | 5 |
| 7. | Diglycerin | 5 |
| 8. | PEG-12 | 15 |
| 9. | Phenoxyethanol | 0.3 |
| 10. | Chili pepper extract | q.s. |
| 11. | Glycerin | Balance |
| | Total | 100.0 |

| | | |
|---|---|---|
| (Note 1) Mixture of 70-80% dimethicone + 20-30% (dimethicone/(PEG-10/15)) crosspolymer, Shin-Etsu Chemical Co., Ltd. (Note 2) (Vinyl dimethicone/methicone silsesquioxane) crosspolymer, Shin-Etsu Chemical Co., Ltd. | | |

### (Production method)

- A:: Ingredients 1 to 4 were mixed uniformly.
- B:: Ingredients 5 to 11 were mixed uniformly.
- C:: The mixture obtained in B was added to the mixture obtained in A and emulsified to give a water-in-oil cream.
The water-in-oil cream obtained as described above had a very good feeling on use and was also excellent in stability without any changes due to temperature or aging.

### [Example 15] Water-breaking W/O foundation

| | Composition | % |
|---|---|---|
| 1. | KSG-270 (Note 1) | 4 |
| 2. | KSG-18A (Note 2) | 1 |
| 3. | Aminoalcohol-modified silicone obtained in Production Example 2 | 0.1 |
| 4. | Ethylhexyl methoxycinnamate | 7.5 |
| 5. | Dimethylpolysiloxane (2 cs) | 5.6 |
| 6. | Cyclopentasiloxane | 4.8 |
| 7. | KF-6048 (Note 3) | 0.3 |
| 8. | KTP-09W (Note 4) | 5.1 |
| 9. | KTP-09Y (Note 4) | 0.58 |
| 10. | KTP-09R (Note 4) | 0.25 |
| 11. | KTP-09B (Note 4) | 0.07 |
| 12. | Hydrophobized microparticulate zinc oxide (Note 5) | 6 |
| 13. | Butylene Glycol | 8 |
| 14. | Sodium citrate | 0.2 |
| 15. | Magnesium sulfate | 1.5 |
| 16. | Water | Balance |
| | Total | 100.0 |

| | | |
|---|---|---|
| (Note 1) Mixture of 75-85% diphenylsiloxyphenyl trimethicone + 15-25% (dimethicone/(PEG-10/15)) crosspolymer, Shin-Etsu Chemical Co., Ltd. (Note 2) Mixture of 80-90% diphenylsiloxyphenyl trimethicone + 10-20% (dimethicone/phenyl vinyl dimethicone) crosspolymer, Shin-Etsu Chemical Co., Ltd. (Note 3) Cetyl PEG/PPG-10/1 dimethicone, Shin-Etsu Chemical Co., Ltd. (Note 4) KF-9909-treated coloring inorganic pigments, W: white, R: red, Y: yellow, B: black, Shin-Etsu Chemical Co., Ltd. (Note 5) Treated with AES-3083, Shin-Etsu Chemical Co., Ltd. | | |

### (Production method)

- Preparation of oil phase:: Ingredients 1 to 5 were mixed uniformly.
- Preparation of aqueous phase:: Ingredients 13 to 16 were mixed uniformly.
- Preparation of paste phase:: Ingredients 8 to 12 were mixed together in ingredients 6 and 7 and dispersed with a three-roll mill.

The aqueous phase was gently added to the oil phase to effect a phase change. After emulsification, the paste was mixed. A water-breaking W/O foundation was thus obtained.

The water-breaking W/O foundation obtained was shown to have a good feeling on use, a good water-breaking feel, and good applicability.

### [Example 16] W/O cosmetic stick

| | Composition | % |
|---|---|---|
| 1. | KSG-330 (Note 1) | 5 |
| 2. | KSG-43 (Note 2) | 5 |
| 3. | Aminoalcohol-modified silicone obtained in Production Example | 9 0.5 |
| 4. | Meadowfoam oil | 2 |
| 5. | KF-56A (Note 3) | 8 |
| 6. | Argania spinosa kernel oil | 0.5 |
| 7. | Ceresin | 10 |
| 8. | Butylene Glycol | 7 |
| 9. | PCA-Na | 2 |
| 10. | Diglycerin | 3 |
| 11. | Sodium citrate | 0.2 |
| 12. | Magnesium sulfate | 1 |
| 13. | Water | Balance |
| | Total | 100.0 |

| | | |
|---|---|---|
| (Note 1) Mixture of 75-85% triethylhexanoin + 15-25% (PEG-15/lauryl dimethicone) crosspolymer, Shin-Etsu Chemical Co., Ltd. (Note 2) Mixture of 65-75% triethylhexanoin + 25-35% (vinyl dimethicone/lauryl dimethicone) crosspolymer, Shin-Etsu Chemical Co., Ltd. (Note 3) Diphenylsiloxyphenyl trimethicone, Shin-Etsu Chemical Co., Ltd. | | |

### (Production method)

- A:: Ingredients 1 to 7 were mixed uniformly at 90°C.
- B:: Ingredients 8 to 13 were mixed uniformly at 85°C.
- C:: The mixture obtained in B was added to the mixture obtained in A and emulsified. The emulsion was poured into a stick container to give a W/O cosmetic stick.
The W/O cosmetic stick obtained had a good feeling on use, good applicability, and excellent emulsion viscosity stability.

## Claims

1. A water-in-oil cosmetic comprising:
(a) an aminoalcohol-modified silicone represented by formula (1) below: 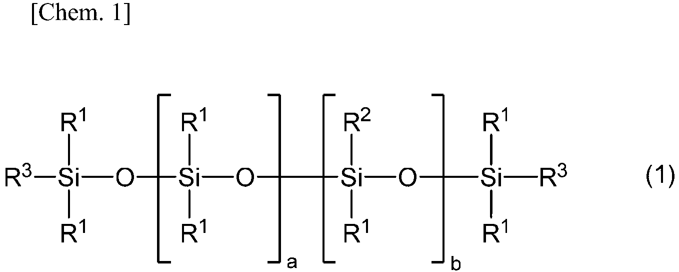
wherein R¹ independently at each occurrence is a monovalent hydrocarbon group selected from C1-C20 alkyl groups, C6-C15 aryl groups, and C7-C15 aralkyl groups;
R² independently at each occurrence is a group represented by formula (2) below: 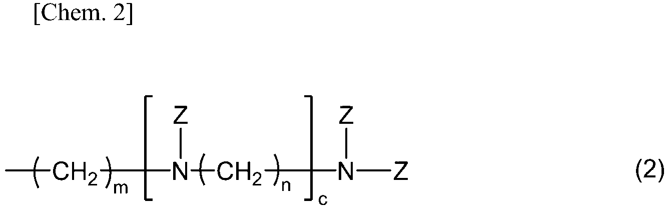
wherein Z independently at each occurrence is a group selected from hydrogen atom, -CH₂CH(OH)CH₃, and -CH(CH₃)CH₂OH, 50 mol% or more of a number of moles of all Zs in the molecule are groups selected from -CH₂CH(OH)CH₃ and -CH(CH₃)CH₂OH, m is an integer of 1 to 10, n is an integer of 1 to 5, and c is an integer of 0 to 4; R³ is R¹ or R²; a is 1 to 100; b is 0 to 10; and when b = 0, at least one of R³s is R², and
(b) 0.6 to 1.8 mass% of an inorganic salt.

2. The water-in-oil cosmetic according to claim 1, wherein in formula (1), R² is selected from aminoalcohol groups represented by formulas (3) and (4) below: 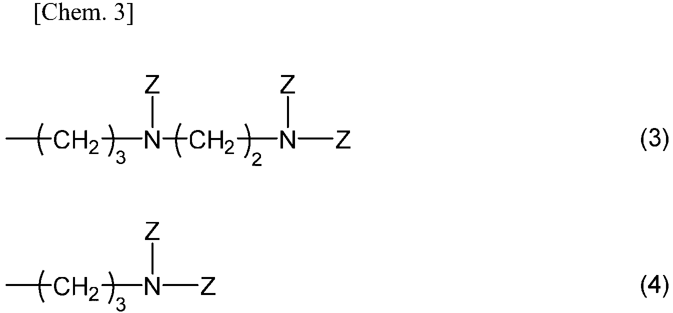 wherein Z is the same as defined above.

3. The water-in-oil cosmetic according to claim 1, wherein in formula (1), b is an integer of 1 to 10, and a/b is 10 to 30.

4. The water-in-oil cosmetic according to claim 1, wherein in formula (1), 99 to 100 mol% of the number of moles of all Zs in the molecule are -CH₂CH(OH)CH₃ or -CH(CH₃)CH₂OH.

5. The cosmetic according to claim 1, wherein the ingredient (a) has a kinematic viscosity of 100 to 3,500 mm²/s.

6. The water-in-oil cosmetic according to claim 1, wherein the ingredient (b) is one or more selected from sodium chloride and magnesium sulfate.

7. The water-in-oil cosmetic according to any one of claims 1 to 6, further comprising a water-soluble polyhydric alcohol (c).
